# EUROPEAN PATENT APPLICATION

(11) **EP 2 573 100 A1**
(43) Date of publication of application: **27.03.2013**
(21) Application number: 11075215.1
(22) Date of filing: 23.09.2011
(51) Int. Cl.: C07H 17/04, A61K 31/702, A61P 31/04

(54) **Total synthesis of the core tetrasaccharide of the lipopolysaccharide from Neisseria meningitidis**

(71) Applicant: Max-Planck-Gesellschaft zur Förderung der Wissenschaften e.V., 80539 München (DE)
(72) Inventor: Seeberger, Peter H., 14532 Kleinmachnow (DE); Yang, You, 12167 Berlin (DE); Anish, Chakkumkal, Dr., 12249 Berlin (DE); Reinhardt, Anika, 12161 Berlin (DE)
(74) Representative: Arth, Hans-Lothar

(57) **Abstract**

The present invention relates to the total synthesis of the α-GlcNAc-(1→2)-α-Hep-(1→3)-α-Hep-(1→5)-α-Kdo core tetrasaccharide of the lipopolysaccharide from *Neisseria meningitidis,* the resulting product obtained by the total synthesis and α-GlcNAc-(1→2)-α-Hep-(1→3)-α-Hep-(1→5)-α-Kdo for use as a vaccine for immunization against diseases caused by infection with bacteria containing the tetrasaccharide, especially for immunization against meningitis, septicaemia, pneumonia and nasopharyngitis caused by *Neisseria meningitidis.*

## Description

### Field of the invention

The present invention relates to the total synthesis of the α-GlcNAc-(1→2)-α-Hep-(1→3)-α-Hep-(1→5)-α-Kdo core tetrasaccharide **1** of the lipopolysaccharide from *Neisseria meningitidis,* the resulting product obtained by the total synthesis and α-GlcNAc-(1-2)-α-Hep-(1-3)-α-Hep-(1-5)-α-Kdo for use as a vaccine for immunization against diseases caused by infection with bacteria containing the tetrasaccharide, especially for immunization against meningitis, septicaemia, pneumonia and nasopharyngitis caused by *Neisseria meningitidis.*

### Background of the invention

Bacterial meningitis is a severe infection of the meninges, and accounts for about 1,200,000 cases of the disease worldwide annually, affecting mostly infants, children, and young adults who do not have specific protective antibodies. *Neisseria meningitidis* is a leading cause of bacterial meningitis and the only Gram-negative encapsulated bacterium responsible for large epidemics of the disease. Rapid development of meningococcal infection often leads to death within a few hours; even with appropriate care at least 10% of patients die, while up to 20% of survivors are left with permanent aftereffects such as deafness, epilepsy, mental retardation, or other neurological disorders.

Two types of polysaccharides, capsular polysaccharide (CPS) and lipopolysaccharide (LPS), are the major virulence factors in *N. meningitidis* infections. Meningococcal CPS consists of a slimy polysaccharide capsule surrounding the bacteria, and is a linear chain of mono or disaccharide repeating units. Based on these CPS structures, *N. meningitidis* can be divided into 13 serogroups. Five of these serogroups (A, B, C, Y, W135) are associated with the vast majority of meningococcal disease, and their distribution is strictly limited geographically. For example, in Europe and North America, most cases of meningitis are caused by bacteria from serogroups B and C, while in the African meningitis belt, where the infection rate ranges from 100 to 800 per 100,000 population during epidemics, group A bacteria are the predominant cause. CPS-based meningococcal vaccines were first introduced in the 1970s and are now commercially available. These vaccines are comprised of CPS isolated from natural sources, assembled as bivalent polysaccharides containing combinations of glycans from A + C, tetravalent polysaccharides combinations of A + C + Y + W 135, as well as the group A glycocojugate, the group C glycoconjugate and the tetravalent groups A + C + Y + W135 glycoconjugate made by chemical conjugation of each polysaccharide to a carrier protein (typically tetanus toxoid or CRM197). Although CPS-based vaccines can provide large-scale protection of public health against most CPS serogroups, there is currently no vaccine to prevent group B infection. This is because the structure of CPS from group B resembles the carbohydrate structure of the human central nervous system and therefore does not qualify as a suitable vaccine candidate due to the risk of triggering an autoimmune response.

Gram-negative bacteria LPS is located in the outer membrane and typically consists of three parts, the core structure, the *O*-antigen, and the lipid A moiety. However, the LPS of *N*. *meningitidis* do not possess a polymeric *O*-antigen, thus, in this case, the core is the most exposed and hence immunodominant part of LPS. All clinically relevant meningococcal strains share a common LPS inner core structure, therefore an LPS-based vaccine may generate broadly protective antibodies against the major disease-causing serotypes of *N*. *meningitidis,* including group B. Over the past two decades, efforts have been focused on developing LPS-based vaccines that would provide populationwide, comprehensive protection against meningococcal disease across serogroups A, C and most importantly group B. The potential for LPS as a candidate vaccine was indicated by the ability of core oligosaccharides isolated from *N*. *meningitidis* to elicit the production of antibodies and provide an effective, protective immune response to *N*. *meningitidis* infections.

However, relying on bacterial isolates limits the availability of LPS to scarce amounts, and the glycans frequently show high degrees of microheterogenicity in the outer core, which complicates structural and biological analysis. Consequently, antibody responses to different batches of isolated lipopolysaccharides can vary. Another complication is that isolated LPS must be detoxified to remove the endotoxin lipid A before it can be considered as a vaccine moiety. To fully explore the effectiveness of LPS oligosaccharides as vaccine candidates against *N. meningitidis,* and to identify the most immunogenic epitopes, it is essential that large amounts of well defined, nontoxic synthetic LPS core structures are available. Despite the great interest in synthetic LPS structures, only a few *N*. *meningitidis* LPS-related oligosaccharides containing L-*glycero*-D-mannoheptose (Hep) and 3-deoxy-D-*manno*-2-octulosonic acid (Kdo), have been synthesized to date. LPS core tetrasaccharide synthesis is particularly difficult because of two synthetic challenges imposed by the LPS core structure: production of Hep building blocks, and generation of the Hep-Kdo disaccharide.

It is an object of the present invention to provide a route for a total synthesis of the α-GlcNAc-(1-2)-α-Hep-(1-3)-α-Hep-(1-5)-α-Kdo core tetrasaccharide of the lipopolysaccharide from *Neisseria meningitidis.* Furthermore, it is an object of the present invention to provide a vaccine for immunization against diseases caused by bacteria containing the α-GlcNAc-(1→2)-α-Hep-(1→3)-α-Hep-(1→5)-α-Kdo tetrasaccharide, especially for providing a vaccine for immunization against meningitis septicaemia, pneumonia and nasopharyngitis caused by *Neisseria meningitidis.*

The object of the present invention is solved by the teaching of the independent claims. Further advantageous features, aspects and details of the invention are evident from the dependent claims, the description, and the examples of the present application.

### Description of the invention

The present invention describes the first total synthesis of the core tetrasaccharide of *N. meningitidis* LPS. To this end, an effective and reliable *de novo* synthetic route was also developed that gives ready access to versatile, differentially-protected heptose building blocks. The convergent [2 + 2] approach used to produce the tetrasaccharide also relied on the construction of a sterically demanding 1 → 5 linked Hep-Kdo disaccharide, a challenge that was met effectively by TMSOTf-promoted glycosylation with heptose trichloroacetimidate. This synthetic strategy may serve more generally as a means to access LPS core structures consisting of heptose and Kdo. As such, this method shall find wide application in the synthesis of larger and more complex LPS structures of Gram-negative bacteria.

The spacer on the reducing terminus of the synthetic LPS core tetrasaccharide **1** can serve as handle for conjugation to a carrier protein. This neoglycoconjugate will be used in subsequent immunology studies to explore the efficacy of LPS as a vaccine.

Thus the present invention relates to the total synthesis of the α-GlcNAc-(1 →2)-α-Hep-(1→3)-α-Hep-(1→5)-α-Kdo tetrasaccharide **1** which has the following chemical formula:

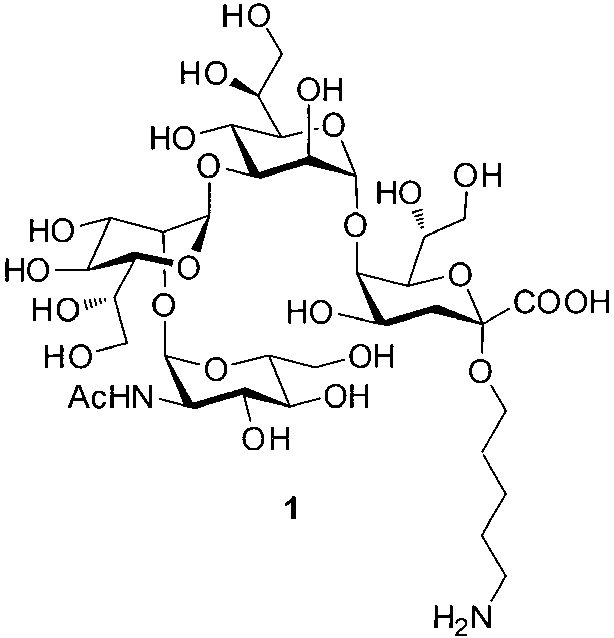

Thus one aspect of the present invention is directed to the total chemical synthesis of the α-GlcNAc-(1→2)-α-Hep-(1→3)-α-Hep-(1→5)-α-Kdo tetrasaccharide 1**.**

Another aspect is directed to the α-GlcNAc-(1→2)-α-Hep-(1→3)-α-Hep-(1→5)-α-Kdo tetrasaccharide **1** obtained by the total chemical synthesis as disclosed herein.

A further aspect of the present invention is related to the α-GlcNAc-(1→2)-α-Hep-(1→3)-α-Hep-(1→5)-α-Kdo tetrasaccharide **1** which is free of any phosphoethanolamine (PEA) which is inevitably present in any extracts or isolates from biological material and especially bacterial isolates. Also this tetrasaccharide **1** is free of any endotoxin lipid A which is present in most extracts or isolates from biological material and especially bacterial isolates. Due to these contaminations the oligosaccharides obtained or derived from natural sources were and could so far not used as vaccines. Moreover such a tetrasaccharide **1** is substantially pure, having a purity of ≥ 95%, preferably ≥ 96%, more preferably ≥ 97%, still more preferably ≥ 98%, and most preferably ≥ 99%. In addition the chemically synthesized tetrasaccharide **1** does not have any microheterogenicity as the oligosaccharides from biological sources do.

Still another aspect of the present invention is directed to a vaccine containing the α-GlcNAc-(1-2)-α-Hep-(1-3)-α-Hep-(1-5)-α-Kdo tetrasaccharide **1** obtained by the total synthesis disclosed herein or containing the tetrasaccharide **1** without any traces of the PEA and endotoxin lipid A. The tetrasaccharide **1** used to prepare the vaccine has a purity of ≥ 95%, preferably ≥ 96%, more preferably ≥ 97%, still more preferably ≥ 98%, and most preferably ≥ 99%. The tetrasaccharide **1** does also not exhibit any microheterogenicity especially in the glycans.

The tetrasaccharide **1** and the pharmaceutical compositions containing the tetrasaccharide **1** and especially the vaccine containing the tetrasaccharide **1** are highly useful for eliciting antibodies, including monoclonal antibodies (MAb). Such elicited antibodies are also used for the treatment of diseases or support the treatment of diseases caused by bacteria containing the tetrasaccharide **1** such as *Neisseria meningitidis.* These antibodies could also be used for diagnostic purposes, for instance, to diagnose diseases caused by bacteria containing the tetrasaccharide **1** such as meningitis, septicemia, pneumonia and nasopharyngitis.

Moreover the tetrasaccharide **1** can be used as a marker in immunological assays for diagnostics of diseases caused by bacteria containing the tetrasaccharide **1**. Such assays comprise, for instance, microarray and ELISA useful for diagnosis of diseases caused by bacteria containing or comprising the tetrasaccharide **1**. Such diseases which can be diagnosed comprise meningitis, septicemia, pneumonia and nasopharyngitis.

The tetrasaccharide **1** itself as well as the pharmaceutical compositions containing the tetrasaccharide **1** and especially vaccines containing the tetrasaccharide **1** are highly useful for the treatment and prophylaxis of diseases caused by bacteria containing the tetrasaccharide **1** such as the group of diseases comprising or consisting of meningitis, septicemia, pneumonia and nasopharyngitis. Thus the tetrasaccharide **1** is preferably used for the preparation of a pharmaceutical composition and especially a vaccine for the treatment and prophylaxis of diseases caused by bacteria such as *Neisseria meningitidis* containing the tetrasaccharide **1** and more preferably for the preparation of a pharmaceutical composition and especially a vaccine for the treatment and prophylaxis of meningitis, septicemia, pneumonia and nasopharyngitis.

The bacteria containing the tetrasaccharide **1** are selected from the group consisting of all strains of *Neisseria meningitidis,* wherein the LPS immunotypes are L1, L2, L3, L4, L5, L6, L7, L8, L9 and/or L11. The LPS immunotypes L1, L2, L3, L4, L5, L6, L7, L8, L9 and L11 contain the tetrasaccharide **1**. The immunotypes L1 - L9, and L11 are associated with serogroups A, B, and C.

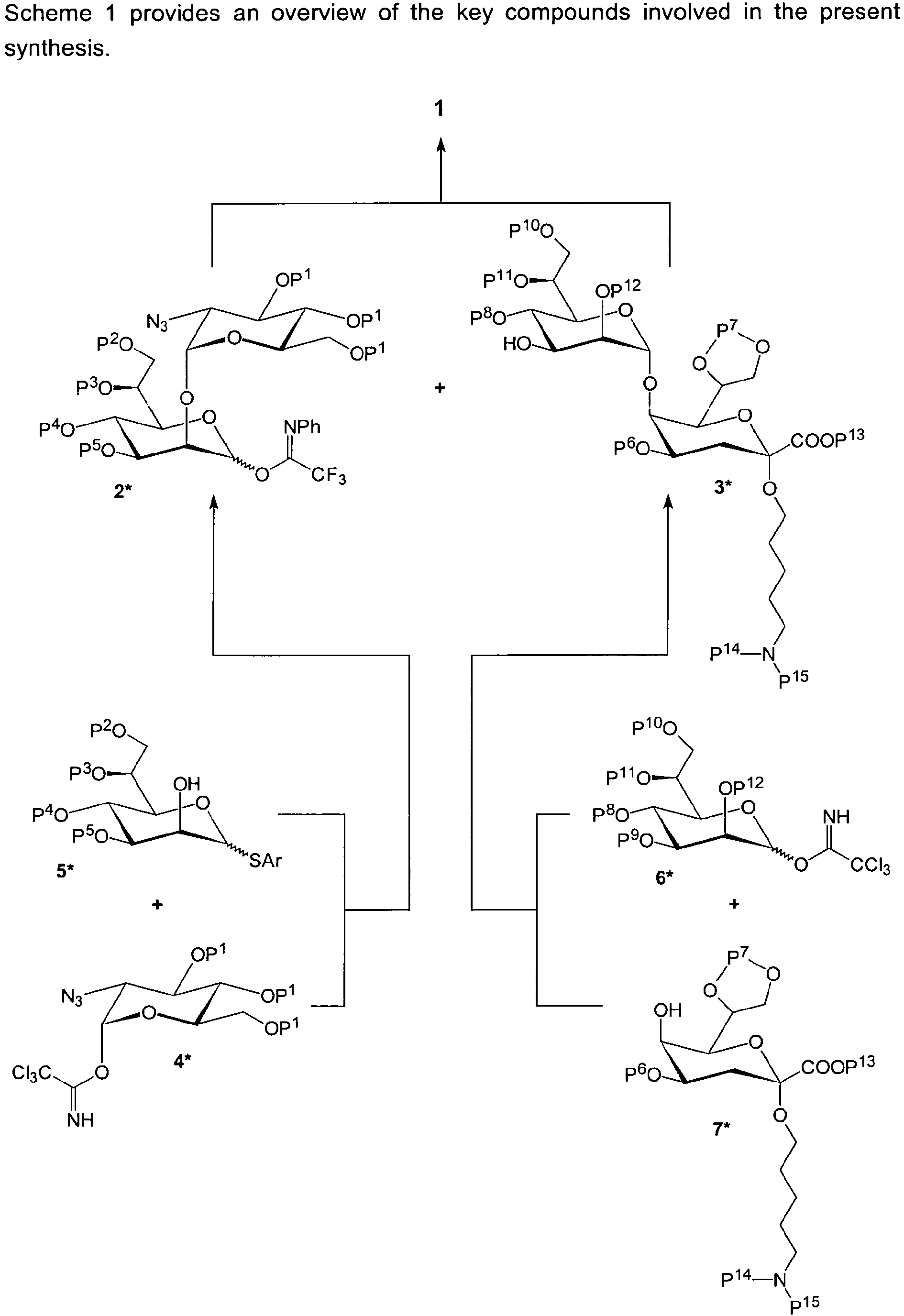

Herein for the first time the total synthesis of the tetrasaccharide **1** is disclosed. The present invention relates to the total synthesis of the tetrasaccharide **1** of the chemical formula: 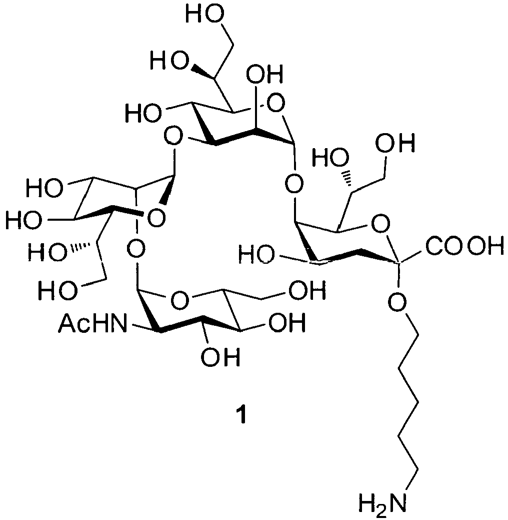 comprising the steps:
A) Reacting the compound **6*** of the formula 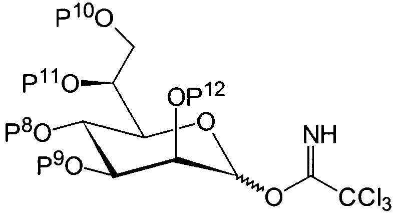 wherein
   P⁸ - P¹² represent protecting groups
   with the compound **7*** of the formula 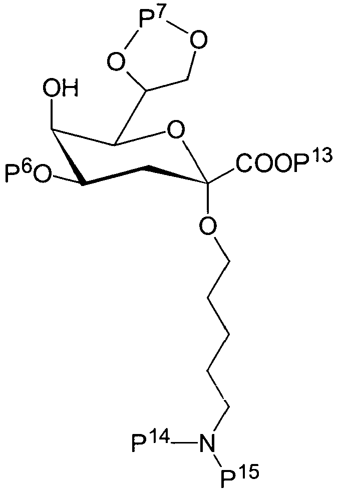 wherein
   P⁶, P⁷, P¹³ - P¹⁵ represent protecting groups
   in order to obtain compound **31*** of the following chemical formula 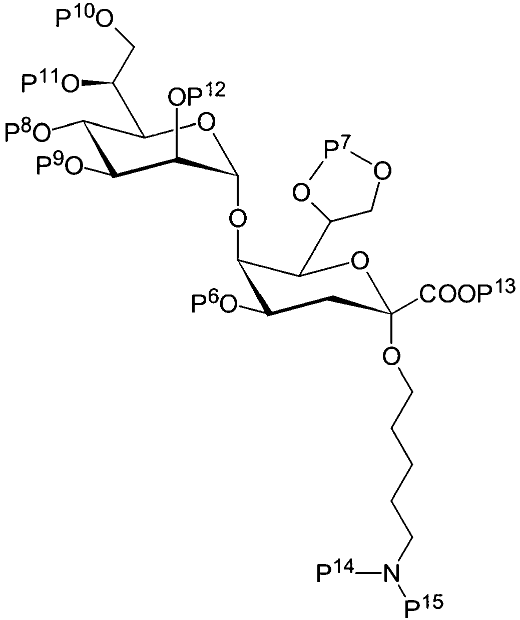 wherein the protecting group P⁹ is selectively cleaved in order to obtain compound **3*.**
B) Reacting the compound **4*** of the formula 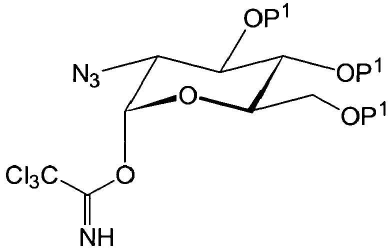 wherein
   the groups P¹ represent the same protecting group with the compound **5*** of the following chemical formula 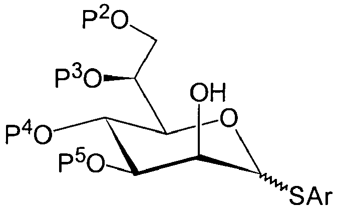 wherein
   P² - P⁵ represent protecting groups and
   Ar represents an aromatic ring or aromatic ring system
   in order to obtain compound **30*** of the following chemical formula 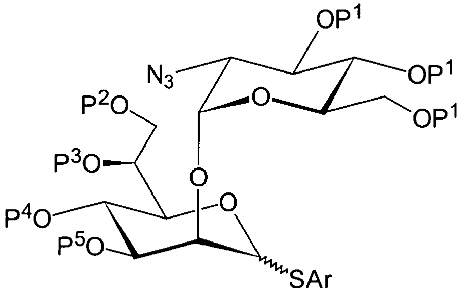 wherein
   the group -SAr is converted to the group -O-C(=NPh)-CF₃ in order to obtain compound **2*** of the following chemical formula 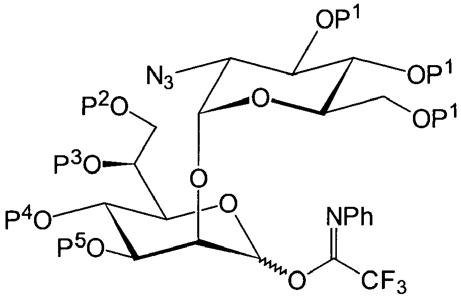
C) Reacting the compound **2*** with compound **3*** of the formula

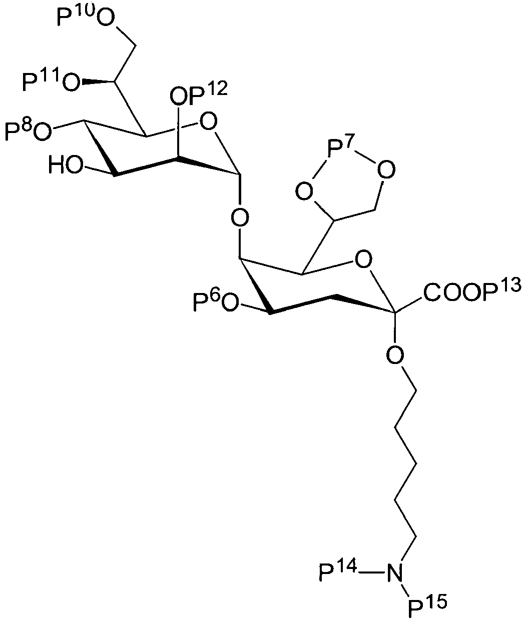

in order to obtain compound **32*** wherein the azide group is converted into an acetamide group and wherein the protecting groups P¹ - P⁸ and P¹⁰ - P¹⁵ are cleaved in order to obtain compound **1**.

Compound **1** is basic and forms salts with various organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, α-toluic acid, (o, m, p)-toluic acid, naphthylamine sulfonic acid, amino acids such as glycone, alanine, valine, leucine, isoleucine, serine, threonine, phenylalanine, tyrosine, tryptophane, lysine, arginine, histidine, asparaginic acid, glutamic acid, asparagines, glutamine, cysteine, methionine, proline, 4-hydroxyproline, N,N,N-trimethyllysine, 3-methylhistidine, 5-hydroxylysine, O-phosphoserine, γ-carboxyglutamate, ε-N-acetyllysine, ω-N-methylarginine, citrulline and ornithine, and other mineral or carboxylic acids well known to those skilled in the art. The salts are prepared by contacting the free base form with a sufficient amount of the desired acid to produce a salt in the conventional manner.

The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

Thus the inventive synthesis may further comprise step D)
D) preparing a salt of compound **1** or preparing a lyophilisate of compound **1** or of the salt of compound **1**.

It is preferred that the reaction of compound **6*** and **7*** is performed in a polar aprotic solvent using trimethylsilyl trifluoromethanesulfonate (TMSOTf). In addition molecular sieve (MS) such as 4A molecular sieve can be used. The reaction temperature is between -20°C and +20°C, preferably the temperature is between -10°C and +10°C and more preferably the temperature is between -5°C and +5°C and most preferably about 0°C.

The reaction is preferably carried out in a polar aprotic solvent such as acetonitrile, nitromethane, sulfolane, dimethylcarbonate, ethylencarbonate, dimethylsulfoxide, ether such as tetrahydrofurane (THF) or diethylether, ketones such as acetone, butanone or pentanone, amides such as dimethylformamide (DMF) or dimethylacetamide, halogenated solvents such as chloroform, methylene chloride, carboxylic acid ester such as acetic acid ethyl ester.

It is preferred that the reaction of compound **4*** and **5*** is performed in an aprotic solvent using trimethylsilyl trifluoromethanesulfonate (TMSOTf). In addition molecular sieve (MS) such as 4Å molecular sieve can be used. The reaction temperature is between -20°C and +20°C, preferably the temperature is between-10°C and +10°C and more preferably the temperature is between -5°C and +5°C and most preferably about 0°C.

The reaction is preferably carried out in a polar aprotic solvent such as acetonitrile, nitromethane, sulfolane, dimethylcarbonate, ethylencarbonate, dimethylsulfoxide, ether such as tetrahydrofurane (THF) or diethylether, ketones such as acetone, butanone or pentanone, amides such as dimethylformamide (DMF) or dimethylacetamide, halogenated solvents such as chloroform, methylene chloride, carboxylic acid ester such as acetic acid ethyl ester.

Preferably the conversion of compound **30*** to compound **2*** is performed in two steps. The first step is reacting compound **30*** in a polar aprotic solvent and water mixture using N-bromosuccinimide (NBS) and the second step involves reacting the product obtained after the first step with CF₃C(=NPh)Cl and a base in a polar aprotic solvent. Both steps are preferably carried out at room temperature or room temperature ± 15°C. For the first step solvent mixtures of a keton and water are preferred such as acetone /water mixtures. For the second step polar aprotic solvents such as acetonitrile, nitromethane, sulfolane, dimethylcarbonate, ethylencarbonate, dimethylsulfoxide, ether such as tetrahydrofurane (THF) or diethylether, ketones such as acetone, butanone or pentanone, amides such as dimethylformamide (DMF) or dimethylacetamide, halogenated solvents such as chloroform, methylene chloride, carboxylic acid ester such as acetic acid ethyl ester are suitable.

Preferably the conversion of compound **31*** to **3*** is performed in a polar aprotic solvent by means of hydrazine or a hydrazinium salt. Suitable polar aprotic solvents are mentioned above. The reaction is preferably conducted at room temperature or room temperature ± 15°C. Hydrazinium salts of weak acids are preferred such as hydrazinium acetate or hydrazinium proprionate.

It is also preferred that the reaction of compound **2*** and the compound **3*** is performed in a polar aprotic solvent using trimethylsilyl trifluoromethanesulfonate (TMSOTf). In addition molecular sieve (MS) such 4A molecular sieve can be used. The reaction temperature is between -20°C and +20°C, preferably the temperature is between -10°C and +10°C and more preferably the temperature is between -5°C and +5°C and most preferably about 0°C.

The reaction is preferably carried out in a polar aprotic solvent such as acetonitrile, nitromethane, sulfolane, dimethylcarbonate, ethylencarbonate, dimethylsulfoxide, ether such as tetrahydrofurane (THF) or diethylether, ketones such as acetone, butanone or pentanone, amides such as dimethylformamide (DMF) or dimethylacetamide, halogenated solvents such as chloroform, methylene chloride, carboxylic acid ester such as acetic acid ethyl ester.

Preferably the conversion of compound **32*** to compound **33*** is performed in a polar aprotic solvent to which a base is added or in a polar aprotic basic solvent using thioacetic acid (AcSH). Suitable polar aprotic solvents are mentioned above. Suitable polar aprotic basic solvents are amines such as pyridine. The reaction is preferably conducted at room temperature or room temperature ± 15°C.

Preferably the conversion of compound **33*** to compound **1** is performed in three steps, first the acid-labile protecting groups are cleaved in a mixture of an acid in water; second the base-labile protecting groups are cleaved in a polar aprotic solvent / water mixture or a polar protic solvent / water mixture using a base; and third the protecting groups sensitive for hydrogenation are cleaved by means of hydrogen and a catalyst. The first step is preferably carried out in a mixture of a weak acid such as formic acid, acetic acid, propionic acid in water at elevated temperature. The reaction temperature is preferably between 50°C and 90°C, more preferably between 60°C and 80°C and most preferably between 65°C and 75°C. For the second step suitable protic solvents are alcohols such as methanol, ethanol, propanol, or isopropanol, which are used in combination with water and probably another polar solvent such as acetonitrile, nitromethane, sulfolane, dimethylcarbonate, ethylencarbonate, dimethylsulfoxide, ether such as tetrahydrofurane (THF) or diethylether, amides such as dimethylformamide (DMF) or dimethylacetamide, halogenated solvents such as chloroform, or methylene chloride. The basic cleavage of the protecting groups is preferably performed at room temperature or room temperature ± 15°C. The third step involves cleavage of the protecting groups sensitive for hydrogenation by the use of hydrogen gas and a catalyst such as palladium on carbon (Pd/C) and palladium hydroxide on carbon (Pd(OH)₂/C). The hydrgenation is preferably performed at room temperature or room temperature ± 15°C. Preferably a solvent mixture of water, a weak acid such as formic acid, acetic acid, or propionic acid and a protic solvent such as alcohols such as methanol, ethanol, propanol, or isopropanol.

This synthesis constitutes a milestone which finally enables production of sufficient amounts of the desired LPS structure to support research towards understanding and designing a vaccine against *N. meningitidis.*

According to the present invention the α-GlcNAc-(1→2)-α-Hep-(1-3)-α-Hep-(1→5)-α-Kdo tetrasaccharide **1** is useful for the manufacture of a pharmaceutical composition for use as vaccine for immunization against diseases caused by bacteria containing or comprising the tetrasaccharide **1,** especially for immunization against meningitis, septicaemia, pneumonia and nasopharyngitis caused by *Neisseria meningitidis.*

Specifically, according to the present invention α-GlcNAc-(1→2)-α-Hep-(1→3)-α-Hep-(1→ 5)-α-Kdo **1** is used as a vaccine for immunization against diseases caused by bacteria containing or comprising the tetrasaccharide **1,** especially for immunization against meningitis, septicaemia, pneumonia and nasopharyngitis caused by *Neisseria meningitidis.*

Example of bacteria containing the α-GlcNAc-(1→2)-a-Hep-(1-3)-α-Hep-(1→5)-α-Kdo tetrasaccharide is *Neisseria meningitidis.* All the strains of *Neisseria meningitidis* wherein the LPS immunotypes are L1. L2, L3, L4, L5, L6, L7, L8, L9 and L11 contain such a tetrasaccharide. The immunotypes L1 - L9, and L11 are associated with serogroups A, B, and C.

Examples of diseases caused by bacteria containing the tetrasaccharide are meningitis, septicemia, pneumonia and nasopharyngitis.

### Pharmaceutical compositions

Vaccines according to the present invention comprise the α-GlcNAc-(1-2)-α-Hep-(1 → 3)-α-Hep-(1→5)-α-Kdo tetrasaccharide **1** or enantiomers, stereoisomeric forms, mixtures of enantiomers, diastereomers, mixtures of diastereomers, hydrates, tautomers, solvates or racemates or pharmaceutically acceptable salts thereof. Especially the tetrasaccharide **1** obtained according to the total synthesis disclosed herein is used in these vaccines. The tetrasaccharide **1** obtained by the total synthesis disclosed herein has a purity of at least 95%, more preferably of at least 96%, still more preferably of at least 97%, still more preferably of at least 98%, and most preferably of at least 99% and does not contain any PEA substituents and endotoxin lipid A and does also not have any microheterogenicity.

The expression tautomer is defined as an organic compound that is interconvertible by a chemical reaction called tautomerization. Tautomerization can be catalyzed preferably by bases or acids or other suitable compounds.

The vaccine may be prepared in the form of a suspension or may be lyophilized. The suspension form may be stored frozen. In the lyophilized form, it is preferable to add one or more stabilizers. Optionally, one or more compounds having adjuvant activity may be added as well. Any conventional stabilizers and adjuvants may be included in a vaccine according to this invention.

Vaccination can be performed at any age. The vaccine many be administered subcutaneously, by spray, by injection, orally, intraocularly, intratracheally or nasally.

Another aspect of the present invention relates to pharmaceutical formulations and pharmaceutical compositions containing the vaccine as an active ingredient, together with at least one pharmaceutical acceptable carrier, excipient, solvent and/or diluents.

Further preferred, the pharmaceutical composition is formulated in the form of a lyophilisate or liquid buffer solution.

The vaccine can also be administered in form of its pharmaceutically active salt optionally using substantially nontoxic pharmaceutically acceptable carrier, excipients, adjuvants or diluents. The vaccine of the present invention is prepared in a conventional solid or liquid carrier or diluents and a conventional pharmaceutically-made adjuvant at suitable dosage level in a known way. The preferred preparations and formulations are in administrable form which is suitable for oral application. These administrable forms, for example, include pills, tablets, film tablets, coated tablets, capsules, powders and deposits. Other than oral administratable forms are also possible. The inventive vaccine may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

The vaccine of the present invention, containing the α-GlcNAc-(1→2)-a-Hep-(1→3)-α-Hep-(1→5)-α-Kdo tetrasaccharide **1** or a derivative or pharmaceutically acceptable salts thereof as an active ingredient will typically be administered in admixture with suitable carrier materials suitably selected with respect to the intended form of administration, i.e. oral tablets, capsules (either solid-filled, semisolid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent of the tetrasaccharide.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the vaccine of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive vaccine containing the α-GlcNAc-(1→2)-α-Hep-(1→3)-α-Hep-(1→5)-α-Kdo tetrasaccharide **1** of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semisolid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1 %.

Techniques for the formulation and administration of the vaccine of the present invention may be found in "Remington's Pharmaceutical Sciences" Mack Publishing Co., Easton PA. A suitable vaccine composition comprising at least α-GlcNAc-(1→2)-α-Hep-(1→3)-α-Hep-(1→5)-α-Kdo and/or pharmaceutically acceptable salts thereof may be a solution of the tetrasaccharide in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of the tetrasaccharide refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

### Total synthesis of tetrasaccharide 1

### Synthesis of tetrasaccharide 1

Traditionally, the synthesis of heptose building blocks relied on the one carbon elongation of mannose building blocks at C-6. Elongation of the carbon skeleton employed toxic heavy metals such as mercury and osmium and often produced inseparable mixtures of diastereomers. Our newly developed *de novo* synthetic route for heptose synthesis is straightforward and simplifies the production of versatile, differentially-protected heptose building blocks (Scheme 2). First, reduction of known ketone **10** with L-selectride gave a regioisomer in 82% yield due to 1,3 migration of the TBS group. Protection of newly formed hydroxyl group in **10** with *para*-bromidebenzyl bromide and sodium hydride in dimethylformamide gave silyl ether **11** in 87% yield. The silyl ether in **11** was replaced by a benzyl ether to afford bisacetal **12** in 91% yield over two steps. Under the influence of CSA, the ketal of **12** was removed selectively in a yield of 84%. The newly formed primary hydroxyl group was protected as a TBDPS ether, and the secondary hydroxyl was masked as a TBS ether, to afford dimethylacetal **13** (73% over three steps). Hydrolysis of **13** with catalytic *para*-toluenesulfonic acid in acetone proceeded smoothly to yield aldehyde **8**. Mukaiyama-type aldol reaction between aldehyde **8** and silyl enolether **9** using MgBr_{2·}OEt₂ as a chelating activator afforded the seven carbon backbone **14** with an excellent 2,3-*anti*-3,4-*syn*-selectivity in a satisfactory 65% yield. Treatment of **13** with trifluoroacetic acid gave lactone **15** as the major product and lactone **16** as the minor product.

Lactone **15** was then reduced with lithium tri-*tert*-butoxyaluminium hydride into diols **17** and **18** in a straightforward manner. Partial migration of the silyl group from C-2 to C-3 was observed and both regioisomers were easily separated by flash chromatography. 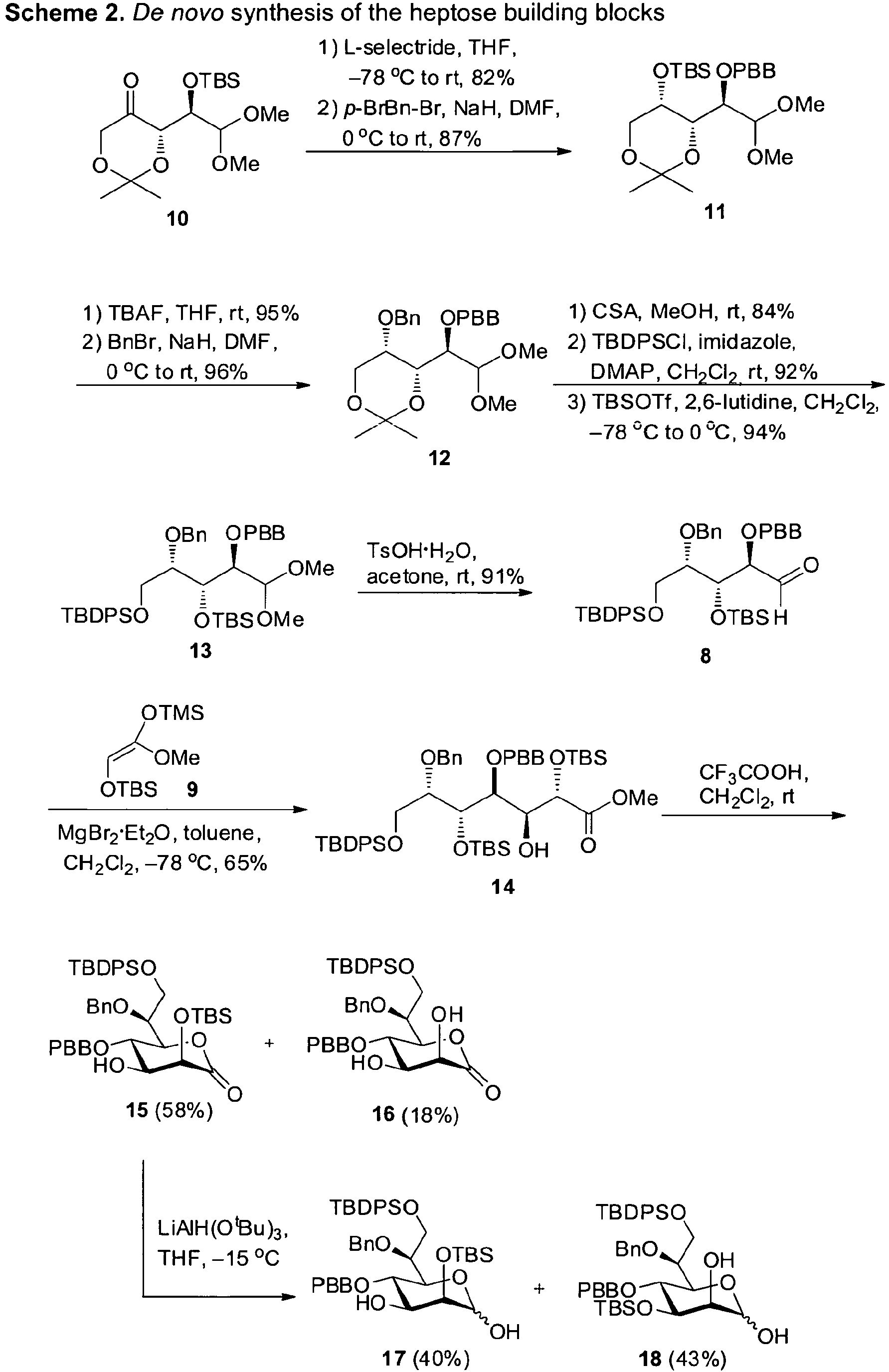 Levulinoylation of both hydroxyl groups present in diol 17 provided diketone 19 in 76% yield (Scheme 3). The silyl groups in 19 were removed with HF in pyridine and subsequent diacetylation gave ester 20 in a yield of 82% over two steps. Ester 20 was then converted into heptose trichloroacetimidate 6 upon selective anomeric delevulinoylation and trichloroacetimidate formation (77% over two steps) or heptose thioglycoside 21 under the agency of boron trifluoride etherate (66%). Diol 18 was diacetylated to diester 22 in an excellent 89% yield. The silyl groups in 22 were replaced by the benzoyl groups analogous to the 19 → 20 conversion, and the resulting anomeric ester was then replaced with 5-tert-butyl-2-methylbenzenethiol to give heptose thioglycoside 23 in 60% yield over three steps. Under the influence of *in situ* hydrogen chloride, the O-acetyl group in **23** was removed to afford heptose building block **5** in 76% yield.

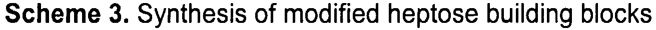

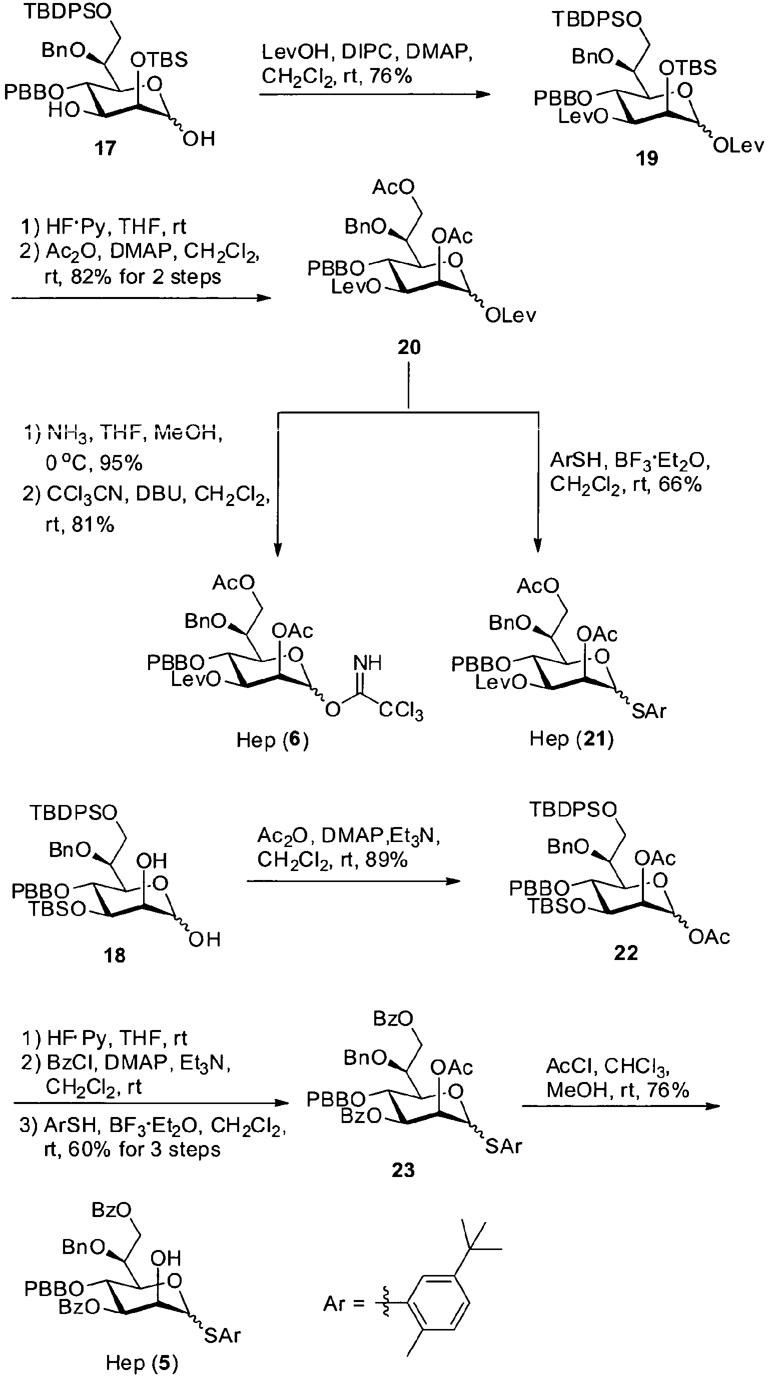

### Synthesis of Kdo building block with an α-linked spacer.

Three different Kdo building blocks **24, 25, 26** and the linker **27** were accessed using modified published methods. In nature, the LPS Kdo moiety of Gram-negative bacteria exists as the α-anomer. Upon activation of the synthetic Kdo building blocks, an elimination reaction to produce the α,β-unsaturated ester frequently occurred due to the presence of the 3-deoxy moiety and the electron-withdrawing C-1 carboxylic function. Therefore stereoselective construction of α-linked Kdo building blocks was dependent on fine tuning of the conditions in the glycosylation reactions. Thus, to find the optimal means to introduce an α-linked spacer into the Kdo moiety, glycosylations of several classes of Kdo building blocks with the linker **27** were briefly examined (Table 1). Linker **27** glycosylation with either glycosyl fluoride **24** or thioglycoside **25** resulted in couplings that produced either β-glycoside or complex mixtures regardless of promoters, solvents and conditions used (entries 1-4, Table 1). Although NIS/TfOH-promoted activation of 2,3-glycal **26** often led to complex mixtures, the coupling of 2,3-glycal **26** with the linker **27** under the catalysis of *in situ* phenylselenyl triflate proceeded smoothly, and the resulting 3-phenylselenium group was reduced with tributyltin hydride to afford α-linked Kdo glycoside **28** in an overall yield of 63% over 2 steps (entries 5 and 6, Table 1).

Removal of the acetyl group in **28** with sodium methoxide, and subsequent regioselective placement of isopropylidene group at the 7,8-vicinal diol using 2-methoxypropene, proceeded smoothly to give **29** (84% over two steps) (Scheme 4). Finally, regioselective benzylation with dibutyltin oxide activation gave the α-Kdo building block **7** containing a free 5-hydroxyl group in 74% yield.

**Table 1. Glycosylation of various Kdo building blocks with the linker 27**

| | | | |
|---|---|---|---|
| 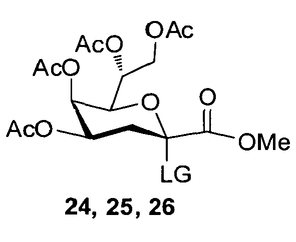 | | 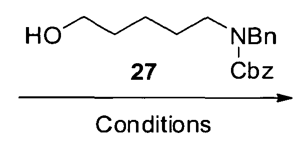 | 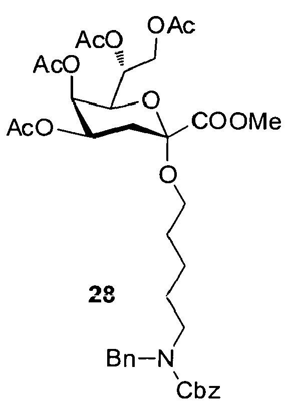 |
| 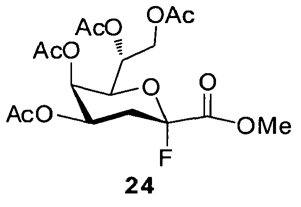 | | 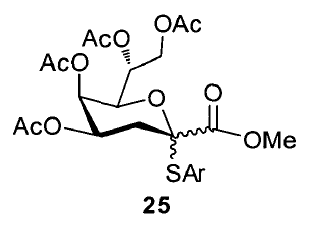 | 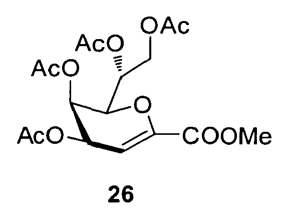 |

| Entry | Kdo | Conditions | Results |
|---|---|---|---|
| 1 | **24** | BF₃OEt₂. Et₃N. CH₂Cl₂. 0 °C | Complex mixture |
| 2 | **25** | NCS. TfOH, CH₂Cl₂, CH₃CN, -65 °C | >80% (β) |
| 3 | **25** | NCS, TfOH, toluene, -65 °C | No reaction |
| 4 | **25** | NIS, TFOH. CH₂Cl₂, rt | Complex mixture |
| 5 | **26** | (a) NIS, TFOH, CH₂Cl₂. rt | Complex mixture |
| | | (b) Bu₃SnH. AIBN, toluene, 110 °C | |
| 6 | **26** | (a) PhSeCl. AgOTf, TMSOTf. CH₂Cl₂. rt | 63% (α) |
| | | (b) Bu₃SnH, AIBN, toluene, 110 °C | |

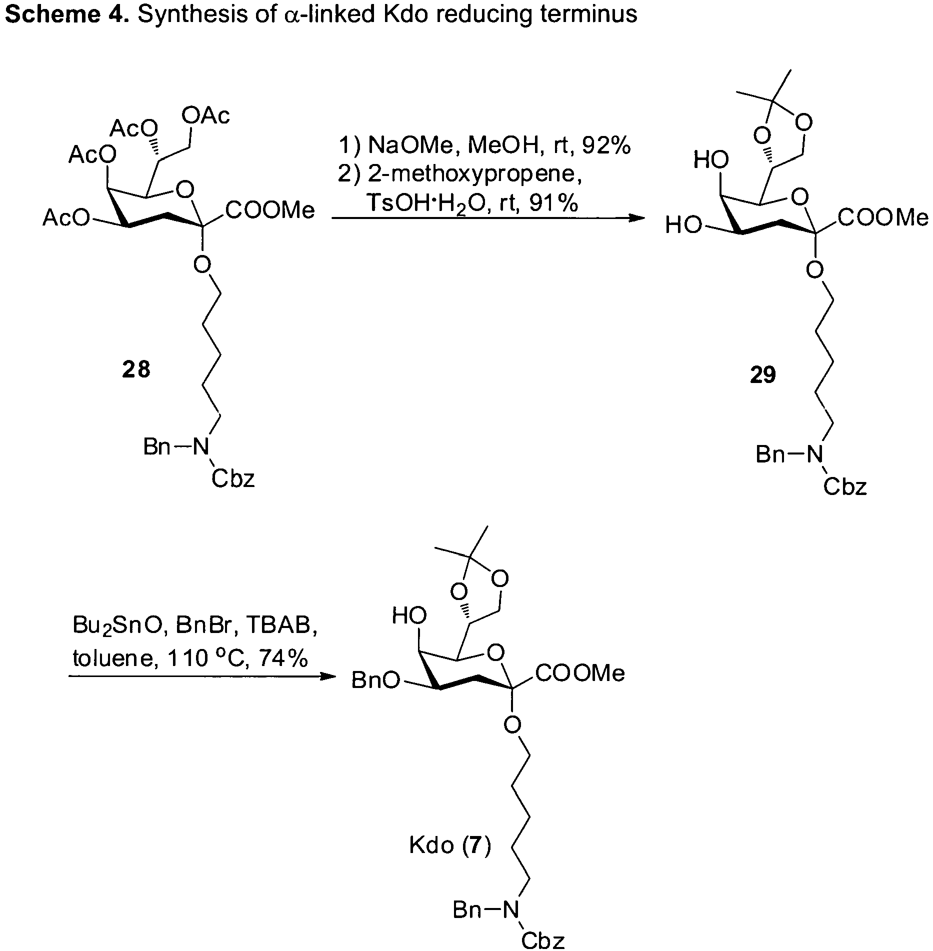

### Synthesis of disaccharide fragments of N. meningitidis LPS.

With the building blocks in hand, efforts were directed at the synthesis of the disaccharide portions, GIcN-Hep and Hep-Kdo of the core tetrasaccharide. Using a catalytic amount of TMSOTf as promoter, coupling of the 2-azidoglucopyranosyl trichloroacetimidate **4** with heptose nucleophile **5** in Et₂O provided the desired α-linked disaccharide **30** in 68% yield, with no detectable production of the β-anomer (Scheme 5). Disaccharide **30** was subjected to selective removal of the anomeric thiol ether with NBS, and subsequent *N*-phenyl trifluoroacetimidate formation, to produce the disaccharide building block **2** over two steps in a yield of 66%.

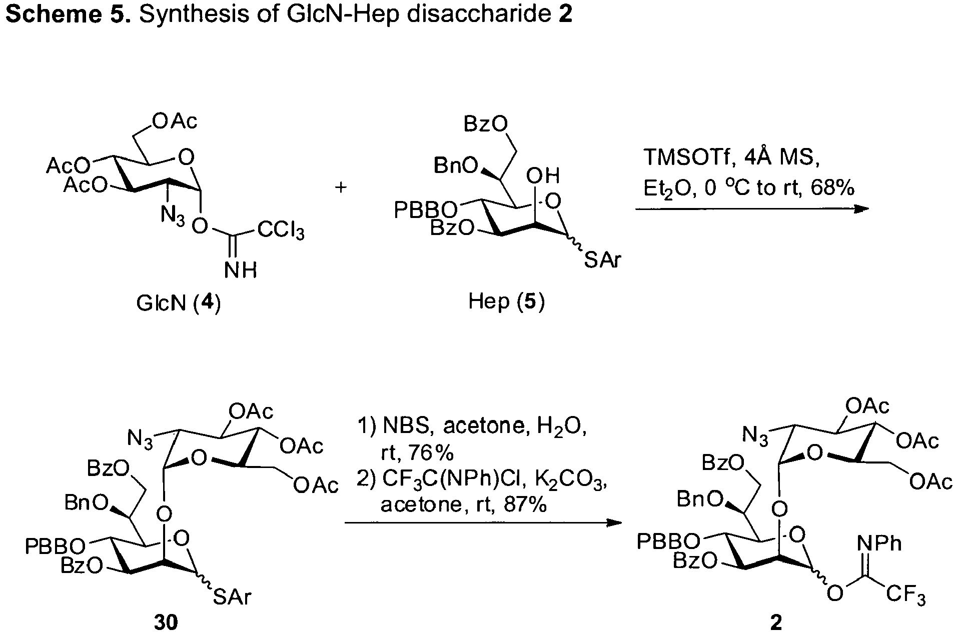

Identification of effective glycosylation conditions for the construction of the 1 → 5 linked Hep-Kdo disaccharide is extremely challenging due to the mismatch and low stereoselectivity in the coupling of the heptose donors with the inert OH-5 position of Kdo building blocks. NIS-mediated glycosylation of heptose thioglycoside **21** with the unreactive Kdo building block **7** or its 5-*O*-stannyl ether led to a hydrolyzed derivative of **21,** and no desired disaccharide product was detected (entries 1 and 2, Table 2). Surprisingly, heptose trichloroacetimidate **6,** activated by catalytic TMSOTf, proved to be superior for glycosylation with Kdo building block **7**. The reaction proceeded efficiently affording the desired α-linked Hep-Kdo disaccharide **31** in a yield of 88% (entry 3, Table 2). It was confirmed that disaccharide **31** had the desired configuration by measuring the coupling constant between heptose C-1 and H-1 (¹*J*_{C-H} = 173.0 Hz). Finally, selective cleavage of the levulinoyl ester of disaccharide **31** with hydrazine acetate afforded disaccharide building block **3** in 73% yield.

**Table 2. Synthesis of Hep-Kdo disaccharide 3**

| | | | | |
|---|---|---|---|---|
| 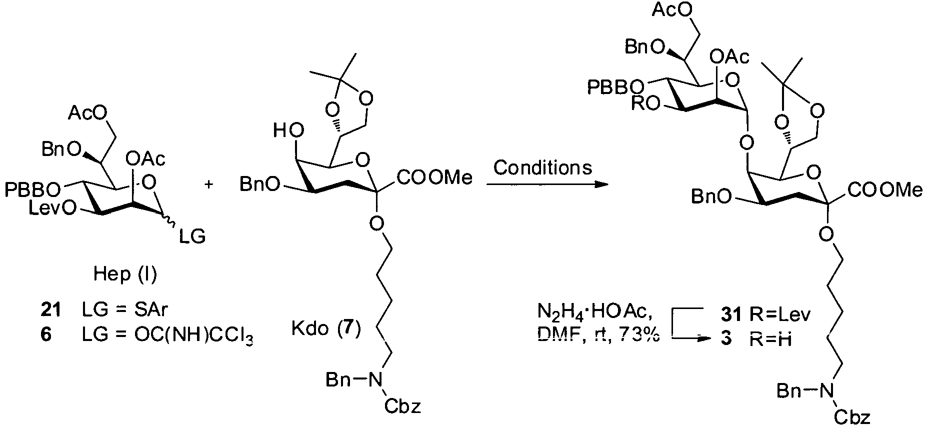 | | | | |

| Entry | Hep | Kdo | Conditions | Results |
|---|---|---|---|---|
| **1** | **21** | 7 | NIS, TMSOTf, 4À MS,CH₂Cl₂, Et₂O, 0 °C to rt | Hydrolyzed |
| 2 | **21** | Bu₃SnO-Kdo | NIS, TMSOTf, 4À MS, CH₂Cl₂, toluene, 0 °C to rt | Hydrolized |
| 3 | **6** | 7 | TMSOTf, 4À MS, CH₂Cl₂, 0°C | 88% |

### Synthesis of N. meningitidis LPS core tetrasaccharide 1.

With the three disaccharide building blocks **30, 2** and **3** in hand, assembly of the tetrasaccharide skeleton of **1** could proceed. Union of thioglycoside **30** and disaccharide **3** gave either complex mixtures or failed regardless of the method of activation (entries 1 and 2, Table 3). Gratifyingly, when disaccharide *N*-phenyl trifluoroacetimidate **2** was used, coupling with disaccharide **3** under the catalysis of TMSOTf in CH₂Cl/Et₂O provided the desired α-linked tetrasaccharide **32** in a satisfactory 72% yield (entry 3, Table 3). The configuration of the newly formed glycosidic linkage was confirmed based on the coupling constants between the C-1 and H-1 of the two corresponding heptoses of the target tetrasaccharide **1** (¹*J*_{C-H} = 169.7 Hz and 172.1 Hz, respectively).

**Table 3. Assembly of tetrasaccharide skeleton 32**

| | | | | |
|---|---|---|---|---|
| 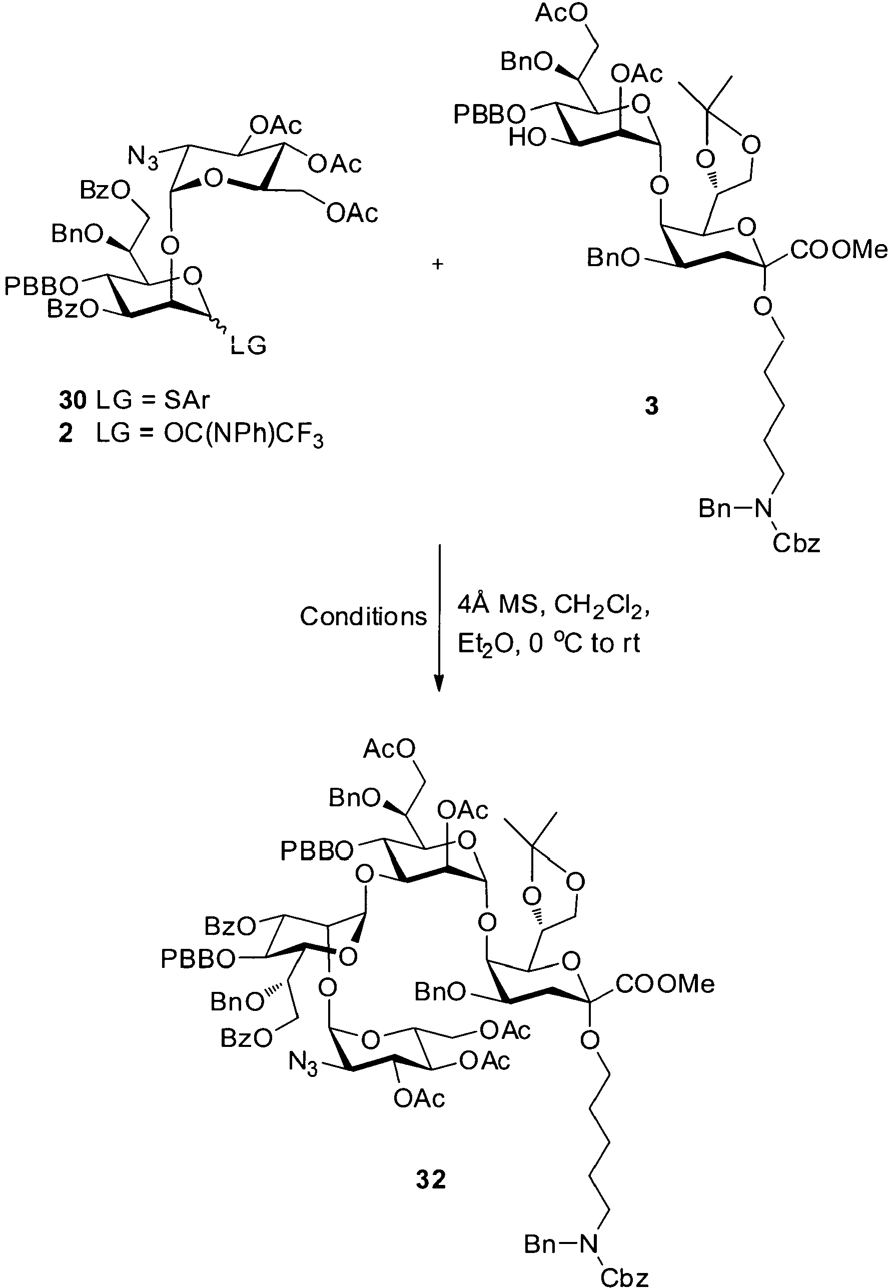 | | | | |

| Entry | GlcN-Hep | Hep-Kdo | Conditions | Results |
|---|---|---|---|---|
| **1** | **30** | **3** | NIS, TMSOTf | Complex mixture |
| 2 | **30** | **3** | DMTST | No reaction |
| 3 | **2** | **3** | TMSOTf | 72% |

The final stage of the synthesis involved the conversion of the azide present in **32** to acetamide **33** (Scheme 6). A one-pot reduction-acetylation sequence using AcSH and pyridine ensured the stability of moderately acid-labile isopropylidene group and a clean reaction. Acetamide **33** was isolated in 91 % yield from azide **32**. Global deprotection of acetamide **33** followed a straightforward three-step procedure. Acidic cleavage of the isopropylidene group, saponification of all of the esters, and hydrogenolysis of the benzyl, *p*-bromobenzyl and benzyloxycarbonyl groups, produced the target tetrasaccharide **1** in a yield of 82% over three steps after final purification on Sephadex LH-20 column.

The structure of the tetrasaccharide **1** was unambiguously confirmed by ¹H, ¹³C and 2D NMR as well as ESI-HRMS. The core tetrasaccharide of *N. meningitidis* LPS, equipped with an amine linker at the reducing end, was thereby obtained by total synthesis.

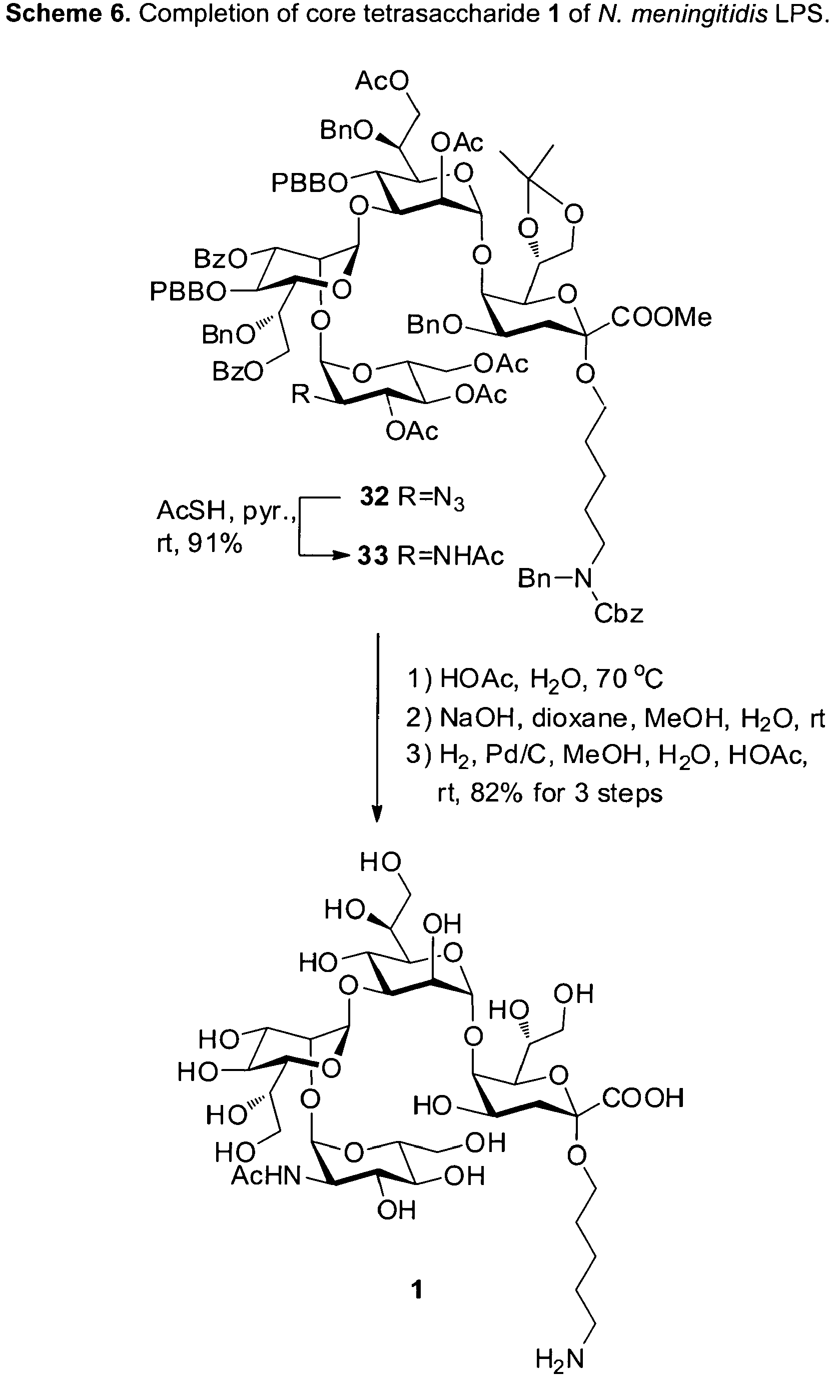

### Description of Figures

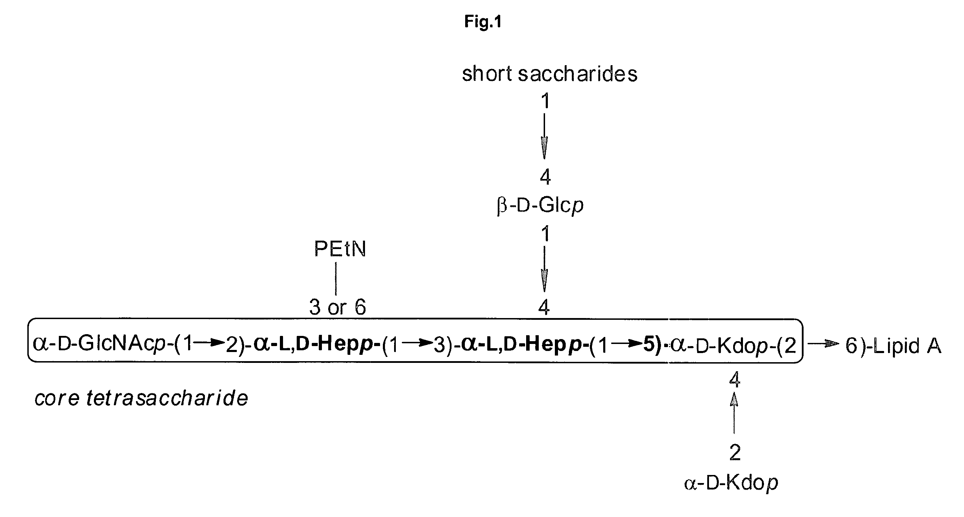

Fig. 1 Schematic showing the structure of *N. meningitidis* LPS

### Examples

**1. General information for chemical synthesis.** Commercial reagents were used without further purification except where noted. Solvents were dried and redistilled prior to use in the usual way. All reactions were performed in oven-dried glassware under an inert atmosphere unless noted otherwise. Analytical thin layer chromatography (TLC) was performed on Kieselgel 60 F254 glass plates precoated with a 0.25 mm thickness of silica gel. The TLC plates were visualized with UV light and by staining with Hanessian solution (ceric sulfate and ammonium molybdate in aqueous sulfuric acid) or sulfuric acid-ethanol solution. Column chromatography was performed on Fluka Kieselgel 60 (230-400 mesh). Optical rotations (OR) were measured with a Schmidt & Haensch UniPol L1000 polarimeter at a concentration (c) expressed in g/100 mL. ¹H and ¹³C NMR spectra were measured with a Varian 400-MR or Varian 600 spectrometer with Me₄Si as the internal standard. NMR chemical shifts (δ) were recorded in ppm and coupling constants (*J*) were reported in Hz. High-resolution mass spectra (HRMS) were recorded with an Agilent 6210 ESI-TOF mass spectrometer at the Freie Universität Berlin, Mass Spectrometry Core Facility.

### 2. Experimental details and characterization data of new compounds

### 2.1. Synthesis of 2,7-di-O-acetyl-3-O-levulinoyl-4-O-para-bromobenzyl-6-O-benzyl-L-glycero-D-manno-heptopyranosyl levulinoate 20

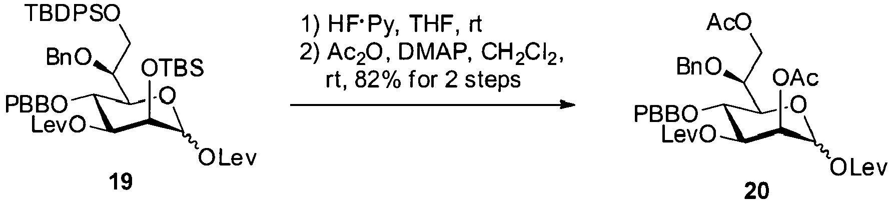

Compound **19** (500 mg, 0.49 mmol) was dissolved in THF (2 mL) at room temperature, followed by addition of 70% HF-pyridine (0.4 mL). After stirring for 2 days, the reaction mixture was carefully quenched with sat. aq. NaHCO₃ and the resulting solution was diluted with EtOAc. The organic layer was washed with brine, dried over Na₂SO₄, filtered and concentrated *in vacuo* to give the corresponding diol as a colorless syrup. To a solution of the above diol and DMAP (12 mg, 0.1 mmol) in CH₂Cl₂ (20 mL), was added Ac₂O (1 mL). After being stirred at room temperature for overnight, the mixture was washed with saturated aqueous NaHCO₃ and brine. The organic layer was dried over Na₂SO₄, filtered, and concentrated *in vacuo.* The residue was purified by silica gel column chromatography (cyclohexane/EtOAc: 1/1) to give ***20*** (300 mg, 82% for 2 steps) as a pale yellow syrup: [α]²⁰_{D} = +53.3 (c 0.3, CHCl₃);¹H NMR (400 MHz, CDCl₃) δ 7.42 - 7.27 (m, 7 H), 7.03 (m, 2 H), 6.06 (d, *J* = 2.4 Hz, 1 H), 5.31 (dd, *J* = 3.6, 9.2 Hz, 1 H), 5.21 (dd, *J* = 2.4, 3.6 Hz, 1 H), 4.80 (d-like, *J* = 12.0 Hz, 1 H), 4.50 (t, *J* = 11.6 Hz, 2 H), 4.44 (dd, *J* = 6.0, 11.6 Hz, 1 H), 4.21 (dd, *J* = 6.0, 11.6 Hz, 1 H), 4.14 (d-like, *J* = 11.6 Hz, 1 H), 4.03 (t, *J* = 9.6 Hz, 1 H), 3.98 (m, 1 H), 3.89 (dd, *J* = 1.6, 9.6 Hz, 1 H), 2.77 - 2.57 (m, 6 H), 2.42 (m, 2 H), 2.17 (s, 3 H), 2.14 (s, 3 H), 2.13 (s, 3 H), 2.04 (s, 3 H); ¹³C NMR (100 MHz, CDCl₃) δ 206.1, 205.9, 171.8, 170.6, 170.2, 169.8, 137.9, 137.0, 131.5, 129.0, 128.5, 128.0, 127.9, 121.5, 90.9, 73.5, 73.4, 73.3, 73.1, 72.4, 72.2, 72.1, 68.5, 62.8, 37.7, 29.7, 29.6, 27.8, 27.7, 20.9, 20.8; HRMS (ESI) *m*/*z* calcd for C₃₅H₄₁BrO₁₃Na [M+Na]⁺ 773.1608, found 773.1645.

### 2.2. Synthesis of 2,7-di-O-acetyl-3-O-levulinoyl-4-O-para-bromobenzyl-6-O-benzyl-1-thio-L-glycero-D-manno-heptopyranosyl trichloroacetimidate 6

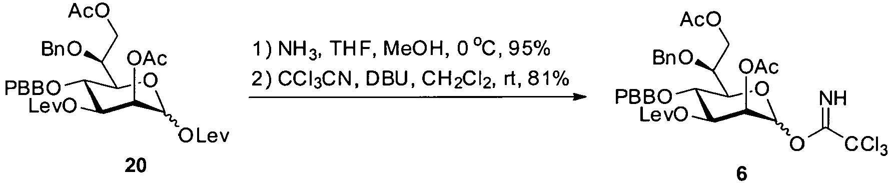

To a solution of compound **20** (170 mg, 0.227 mmol) in THF and methanol (7:3, 10 mL) at 0 °C, was bubbled through gaseous ammonium at a modest rate. After stirring for 30 min at 0 °C, the solution was evaporated *in vacuo* to give a residue, which was purified by silica gel column chromatography (CH₂Cl₂/MeOH: 20/1) to afford the corresponding hemiacetal (140 mg, 95%) as a colorless syrup. To a solution of the above hemiacetal (140 mg, 0.215 mmol) in CH₂Cl₂ (5 mL) was added CCl₃CN (107 µL, 1.07 mmol) and DBU (7 µL, 0.046 mmol). After being stirred at room temperature for 2 h, TLC revealed almost complete conversion of the starting material. The solution was concentrated *in vacuo* to a residue, which was purified by silica gel column chromatography (cyclohexane/EtOAc: 2/1) to give **6** (138 mg, 81%) as a colorless syrup: ¹H NMR (400 MHz, CDCl₃) δ 8.72 (s, 1 H), 7.44 - 7.30 (m, 7 H), 7.06 (d, J = 8.4 Hz, 1 H), 6.28 (d, J = 2.0 Hz, 1 H), 5.44 (dd, J = 2.0, 3.2 Hz, 1 H), 5.40 (dd, J = 3.2, 9.2 Hz, 1 H), 4.82 (d-like, J = 12.0 Hz, 1 H), 4.56 (d-like, J = 11.6 Hz, 1 H), 4.47 (m, 2 H), 4.17 (m, 3 H), 4.02 (m, 2 H), 2.68 (m, 2 H), 2.45 (m, 2 H), 2.18 (s, 3 H), 2.16 (s, 3 H), 2.01 (s, 3 H); LRMS (ESI) m/z calcd for C₃₂H₃₅BrCl₃NO₁₁Na [M+Na]⁺ 816.0, found 815.9.

### 2.3. Synthesis of 5-tert-butyl-2-methylphenyl 2,7-di-O-acetyl-3-O-levulinoyl-4-O-para-bromobenzyl-6-O-benzyl-1-thio-L-glycero-D-manno-heptopyranoside 21

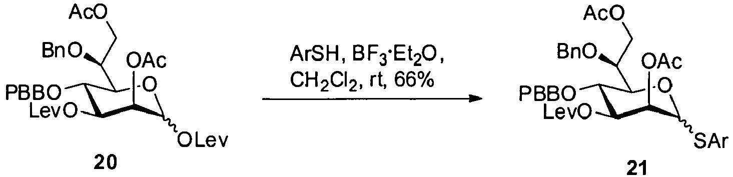

To a solution of compound 20 (50 mg, 0.067 mmol) in CH₂Cl₂ (2.5 mL), was added 5-*tert*-butyl-2-methylbenzenethiol (61 µL, 0.33 mmol) and BF₃OEt₂ (18 µL, 0.14 mmol). After being stirred at room temperature for overnight, the mixture was quenched with Et₃N and concentrated *in vacuo.* The residue was purified by silica gel column chromatography (cyclohexane/EtOAc: 3/1) to give 21 (36 mg, 66%) as a colorless syrup: [OC]²⁰_{D} = +98.8 (c 1.0, CHCl₃); ¹H NMR (400 MHz, CDCl₃) δ 7.46 - 7.29 (m, 8 H), 7.18 (dd, J = 2.0, 8.0 Hz, 1 H), 7.07 (m, 3 H), 5.54 (d, J = 2.0 Hz, 1 H), 5.51 (dd, J = 2.0, 3.2 Hz, 1 H), 5.37 (dd, J = 3.2, 9.2 Hz, 1 H), 4.79 (d-like, J = 11.6 Hz, 1 H), 4.54 (d-like, *J* = 12.0 Hz, 1 H), 4.50 (d-like, *J* = 12.0 Hz, 1 H), 4.44 (dd, *J* = 6.0, 11.2 Hz, 1 H), 4.27 (dd, *J* = 1.2, 9.6 Hz, 1 H), 4.18 (d-like, *J* = 12.0 Hz, 1 H), 4.09 (t, *J* = 9.6 Hz, 1 H), 4.06 (m, 1 H), 3.99 (m, 1 H), 2.69 (m, 2 H), 2.44 (m, 2 H), 2.36 (s, 3 H), 2.17 (s, 3 H), 2.16 (s, 3 H), 1.91 (s, 3 H), 1.29 (s, 9 H); ¹³C NMR (100 MHz, CDCl₃) δ 206.1, 171.8, 170.4, 170.1, 150.0, 137.8, 137.1, 135.8, 132.1, 131.5, 130.1, 128.9, 128.5, 128.1, 128.0, 127.8, 124.9, 121.5, 85.3, 73.5, 73.4, 73.0, 72.9, 72.4, 72.3, 71.6, 62.6, 37.7, 34.5, 31.3, 29.8, 27.8, 26.9, 21.0, 20.7, 20.2; HRMS (ESI) *m*/*z* calcd for C₄₁H₄₉BrSO₁₀Na [M+Na]⁺ 837.2107, found 837.2085.

### 2.4. Synthesis of 5-tert-butyl-2-methylphenyl 2-O-acetyl-3,7-di-O-benzoyl-4-O-para-bromobenzyl-6-O-benzyl-1-thio-L-glycero-D-manno-heptopyranoside 23

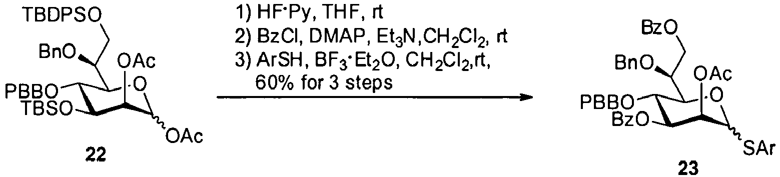

Compound **22** (930 mg, 1.03 mmol) was dissolved in THF (4 mL) at room temperature, followed by addition of 70% HF-pyridine (0.8 mL). After stirring for 2 days, the reaction mixture was carefully quenched with sat. aq. NaHCO₃ and the resulting solution was diluted with EtOAc. The organic layer was washed with brine, dried over Na₂SO₄, filtered and concentrated *in vacuo* to give the corresponding diol as a colorless syrup. To a solution of the above diol and DMAP (200 mg, 1.64 mmol) in CH₂Cl₂ (20 mL), was added Et₃N (2.0 mL) and benzoyl chloride (1.0 mL). After being stirred at room temperature for overnight, the mixture was concentrated *in vacuo* to give a residue, which was purified by silica gel column chromatography (cyclohexane/EtOAc: 5/1 to 3/1) to give the corresponding ester (709 mg) as a white solid. To a solution of the above ester (709 mg, 0.93 mmol) and freshly activated 4Å MS in CH₂Cl₂ (20 mL), was added 5-*tert*-butyl-2-methylbenzenethiol (0.98 mL, 5.31 mmol) and BF₃OEt₂ (0.73 mL, 5.77 mmol). After being stirred at room temperature for overnight, the mixture was quenched with Et₃N and concentrated *in vacuo.* The residue was purified by silica gel column chromatography (cyclohexane/EtOAc: 10/1 to 8/11 to provide **23** (531 mg, α/β = 4.0, 60% for 3 steps) as a white foam: [α] ²⁰_{D} α-anomer: +64.0 (*c* 0.3, CHCl₃); β-anomer: +52.3 (*c* 1.5, CHCl₃); ¹H NMR (400 MHz, CDCl₃) α-anomer: δ 7.88 - 7.81 (m, 4 H), 7.51 - 7.15 (m, 14 H), 7.06 (dd, *J* = 2.0, 8.0 Hz, 1 H), 6.96 - 6.81 (m, 3 H), 5.59 (m, 3 H), 4.82 (d-like, *J* = 11.6 Hz, 1 H), 4.70 (dd, *J* = 5.6, 10.8 Hz, 1 H), 4.51 (d-like, *J* = 12.0 Hz, 1 H), 4.42 (m, 2 H), 4.18 (m, 4 H), 2.26 (s, 3 H), 2.09 (s, 3 H), 1.20 (s, 9 H); β-anomer: δ 8.07 - 7.92 (m, 4 H), 7.61 - 7.36 (m, 14 H), 7.14 (dd, *J* = 2.0, 8.0 Hz, 1 H), 7.04 (m, 3 H), 5.78 (dd, *J* = 2.0, 3.2 Hz, 1 H), 5.63 (d, *J* = 2.0 Hz, 1 H), 5.51 (dd, *J* = 3.2, 9.6 Hz, 1 H), 4.92 (d-like, *J* = 11.6 Hz, 1 H), 4.80 (dd, *J* = 6.0, 10.8 Hz, 1 H), 4.52 (m, 2 H), 4.47 (dd, *J* = 1.6, 9.6 Hz, 1 H), 4.32 (m, 3 H), 4.28 (m, 1 H), 2.38 (s, 3 H), 1.91 (s, 3 H), 1.25 (s, 9 H); ¹³C NMR (100 MHz, CDCl₃) α-anomer: δ 169.8, 165.9, 165.2, 149.9, 137.8, 136.7, 135.5, 133.4, 133.0, 132.2, 131.3, 130.1, 129.6, 129.5, 129.4, 129.3, 128.8, 128.5, 128.4, 128.3, 128.1, 128.0, 127.3, 124.7, 121.5, 85.2, 73.6, 73.5, 73.4, 72.8, 72.6, 72.3, 72.1, 62.3, 34.5, 31.3, 21.0, 20.1; β-anomer: δ 169.8, 166.0, 165.4, 149.9, 138.0, 137.0, 136.1, 133.4, 133.1, 132.1, 131.5, 130.1, 129.9, 129.6, 129.5, 128.7, 128.5, 128.4, 128.3, 127.8, 127.7, 125.0, 121.5, 85.5, 74.0, 73.6, 73.3, 72.6, 72.5, 72.4, 72.3, 62.6, 34.5, 31.3, 20.8, 20.3; HRMS (ESI) *m*/*z* calcd for C₄₈H₄₉BrSO₉Na [M+Na]⁺ 905.2158, found 905.2164.

### 2.5. Synthesis of 5-tert-butyl-2-methylphenyl 3,7-di-O-benzoyl-4-O-para-bromobenzyl-6-O-benzyl-1-thio-L-glycero-D-manno-heptopyranoside 5

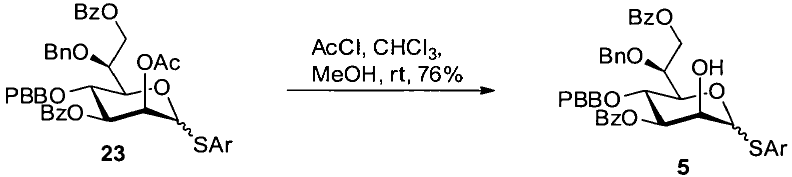

To a solution of thioglycoside **23** (220 mg, 0.249 mmol) in MeOH/CHCl₃ (5/2, v/v, 12.3 mL), was added acetyl chloride (0.53 mL). After being stirred at room tempearature for 1 d, the mixture was diluted with CH₂Cl₂, washed with saturated aqueous NaHCO₃, and brine. The organic layer was dried over Na₂SO₄, filtered, and concentrated *in vacuo.* The residue was purified by silica gel column chromatography (hexane/EtOAc: 6/1) to afford **5** (160 mg, 76%) as a white solid: [α]²⁰_{D} = +79.1 (c 1.0, CHCl₃); ¹H NMR (400 MHz, CDCl) δ 8.02 - 7.83 (m, 4 H), 7.58 - 7.21 (m, 14 H), 7.10 (dd, *J* = 2.0, 8.0 Hz, 1 H), 7.01 - 6.87 (m, 3 H), 5.67 (d, *J* = 2.0 Hz, 1 H), 5.56 (dd, *J* = 3.2, 9.2 Hz, 1 H),4.85 (d-like, *J* = 12.0 Hz, 1 H), 4.76 (dd, *J* = 6.0, 11.2 Hz, 1 H), 4.54 (d-like, *J* = 11.6 Hz, 1 H), 4.46 (m, 3 H), 4.28 (m, 3 H), 4.19 (m, 1 H), 2.30 (s, 3 H), 1.27 (s, 9 H); ¹³C NMR (100 MHz, CDCl₃) δ 165.9, 165.5, 149.9, 137.7, 136.8, 134.9, 133.5, 133.0, 132.7, 131.3, 130.0, 129.7, 129.6, 129.5, 129.0, 128.5, 128.2, 128.1, 128.0, 126.4, 124.3, 121.5, 86.9, 73.7, 73.5, 73.1, 72.6, 72.2, 71.4, 62.4, 34.5, 31.3, 20.1; HRMS (ESI) *m*/*z* calcd for C₄₆H₄₇BrSO₈Na [M+Na]⁺ 863.2052, found 863.2017.

### 2.6. Synthesis of methyl (N-benzyl-benzyloxycarbonyl-5-aminopentyl 4,5,7,8-tetra-O-acetyl-3-deoxy-α-D-manno-oct-2-ulopyranosid)onate 28

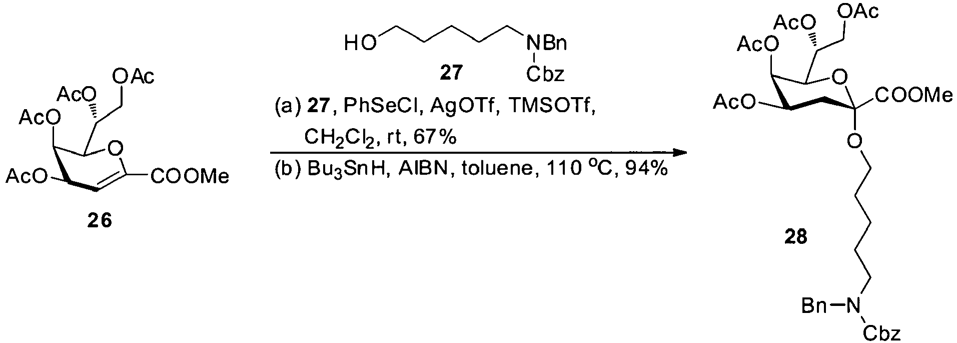

To a stirred solution of phenylselenyl chloride (2.73 g, 14.3 mmol) in CH₂Cl₂ (50 mL) was added AgOTf (2.55 g, 9.92 mmol) and TMSOTf (0.16 mL, 0.86 mmol). After stirring at room temperature for 30 min, a solution of glycal **26** (2.85 g, 7.08 mmol) and linker **27** (3.25 g, 9.92 mmol) in CH₂Cl₂ (70 mL) was added dropwise. After being stirred at room temperature for 2 h, the mixture was diluted with CH₂Cl₂, washed with saturated aqueous NaHCO₃, and brine. The organic layer was dried over Na₂SO₄, filtered and concentrated *in vacuo.* The residue was purified by silica gel column chromatography (cyclohexane/EtOAc: 3/1) to give a white solid (4.2 g, 67%). To a solution of the above solid (4.2 g, 4.75 mmol) in toluene (140 mL), was added tri-*n-*butyltin hydride (3.8 mL, 14.24 mmol) and AIBN (779 mg, 4.75 mmol). After being refluxed for 1.5 h, the mixture was concentrated *in vacuo* and purified by silica gel column chromatography (cyclohexane/EtOAc: 5/2 to 2/1) to afford **28** (3.26 g, 94%) as a colorless syrup: [α]²⁰_{D} = +43.5 (c 0.4, CHCl₃); ¹H NMR (400 MHz, CDCl₃) δ 7.29 - 7.11 (m, 10 H), 5.29 (br s, 1 H), 5.24 (m, 1 H), 5.13 (m, 3 H), 4.51 (d-like, *J* = 12.0 Hz, 1 H), 4.43 (br s, 2 H), 4.05 (dd, *J* = 3.6, 12.4 Hz, 1 H), 3.98 (m, 1 H), 3.71 (s, 3 H), 3.37 (m, 1 H), 3.16 (m, 3 H), 2.09 (dd, *J* = 4.8, 13.2 Hz, 1 H), 2.00 (s, 3 H), 1.95 (m, 1 H), 1.91 (s, 6 H), 1.90 (s, 3 H), 1.47 (m, 4 H), 1.22 (m, 2 H); ¹³C NMR (100 MHz, CDCl₃) δ 170.4, 170.3, 169.9, 169.6, 167.8, 137.8, 128.5, 128.4, 127.9, 127.8, 127.3, 127.2, 98.8, 68.1, 67.6, 67.1, 66.4, 64.3, 63.8, 62.0, 52.6, 32.1, 29.7, 29.2, 23.4, 20.8, 20.7, 20.6; HRMS (ESI) *m*/*z* calcd for C₃₇H₄₇NO₁₄Na [M+Na]⁺ 752.2894, found 752.2921.

### 2.7. Synthesis of methyl (N-benzyl-benzyloxycarbonyl-5-aminopentyl 4-O-benzyl-7,8-O-isopropylidene-3-deoxy-α-D-manno-oct-2-ulopyranosid)onate 7

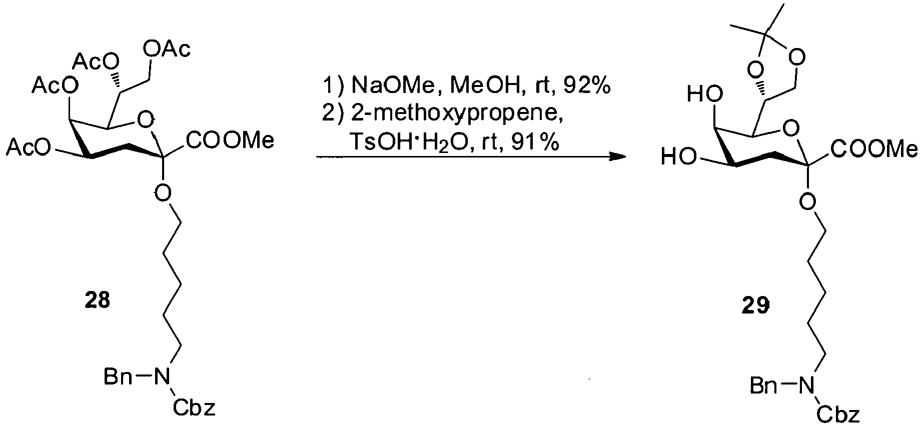

To a stirred solution of compound **28** (1 g, 1.37 mmol) in MeOH (25 mL) was added NaOMe (74 mg, 1.37 mmol). The mixture was stirred at room temperature for 4 h, and then neutralized with Amberlite IR120 H⁺ resin. Filtration, concentration *in vacuo,* and purification by silica gel column chromatography (CH₂Cl₂/MeOH: 12/1) gave the corresponding alcohol (710 mg, 92%) as a white solid. To a stirred solution of the above alcohol (500 mg, 0.89 mmol) in DMF (9 mL), was added 2-methoxypropene (153 µL, 1.60 mmol) and *p*-toluenesulfonic acid monohydrate (40 mg, 0.21 mmol). The mixture was stirred at room temperature for 2 h, and then neutralized with sodium hydrogencarbonate. Filtration, concentration *in vacuo,* and purification by silica gel column chromatography (CH₂Cl₂/MeOH: 40/1) afforded **29** (490 mg, 91 %) as a colorless syrup: [δ]²⁰_{D} = +30.3 (c 0.5, CHCl₃); ¹H NMR (400 MHz, CDCl₃) δ 7.29 - 7.10 (m, 10 H), 5.09 (d-like, *J* = 12.0 Hz, 2 H), 4.42 (br s, 2 H), 4.32 (m, 1 H), 4.07 (dd, *J* = 6.0, 8.4 Hz, 1 H), 3.97 (m, 1 H), 3.88 (m, 2 H), 3.67 (s, 3 H), 3.42 (m, 1 H), 3.31 (m, 1 H), 3.15 (m, 3 H), 2.05 (dd, *J* = 4.8, 12.8 Hz, 1 H), 1.78 (t, *J* = 12.0 Hz, 1 H), 1.44 (m, 4 H), 1.32 (s, 3 H), 1.29 (s, 3 H), 1.18 (m, 2 H); ¹³C NMR (100 MHz, CDCl₃) δ 168.7, 137.8, 128.5, 128.4, 127.9, 127.8, 127.3, 127.2, 109.4, 99.0, 73.6, 72.8, 67.2, 67.1, 66.7, 65.7, 63.6, 52.5, 35.0, 29.7, 29.2, 26.9, 25.3; HRMS (ESI) *m*/*z* calcd for C₃₂H₄₃NO₁₀Na [M+Na]⁺ 624.2785, found 624.2736.

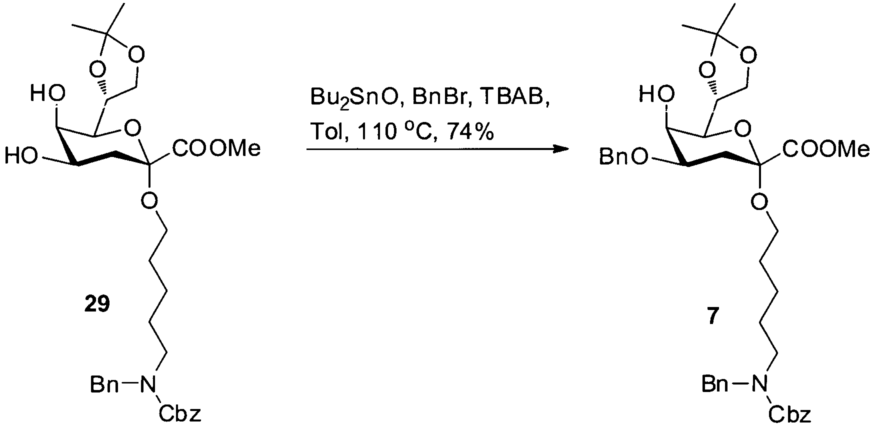

A mixture of compound **29** (490 mg, 0.81 mmol), dibutyltin oxide (304 mg, 1.22 mmol) and 4A MS (500 mg) in toluene (15 mL) was heated at 110 °C for 2 h. After cooling to room temperature, benzyl bromide (0.17 mL, 1.47 mmol) and tetrabutylammonium bromide (158 mg, 0.49 mmol) were added, and the mixture was heated at 110 °C for overnight. The cooling mixture was then filtered and the filrate was evaporated. The residue was dissolved in CH₂Cl₂ and washed with water. The organic layer was dried over Na₂SO₄, filtered and concentrated *in vacuo* to give a residue, which was purified by silica gel column chromatography (cyclohexane/EtOAc: 4/1) to give a syrup, which was dissolved in MeOH (8 mL) and treated with NaOMe (24 mg, 0.44 mmol). The mixture was stirred at room temperature for 3 h, and then neutralized with Amberlite IR120 H⁺ resin. Filtration, concentration *in vacuo,* and purification by silica gel column chromatography (cyclohexane/EtOAc: 3/1) provided **7** (418 mg, 74%) as a colorless syrup: [α]²⁰_{D} = +21.7 (c 0.8, CHCl₃); ¹H NMR (400 MHz, CDCl₃) δ 7.31 - 7.12 (m, 15 H), 5.12 (d-like, *J* = 13.2 Hz, 2 H), 4.53 (dd, *J* = 11.6, 16.4 Hz, 2 H), 4.41 (m, 3 H), 4.09 (m, 2 H), 3.92 (dd, *J* = 4.8, 8.8 Hz, 1 H), 3.85 (m, 1 H), 3.69 (s, 3 H), 3.42 (m, 1 H), 3.34 (m, 1 H), 3.20 (m, 3 H), 2.16 (dd, *J* = 4.8, 12.8 Hz, 1 H), 1.93 (t, *J* = 12.8 Hz, 1 H), 1.48 (m, 4 H), 1.35 (s, 3 H), 1.32 (s, 3 H), 1.20 (m, 2 H); ¹³C NMR (100 MHz, CDCl₃) δ 168.6, 137.9, 137.7, 128.5, 128.4, 127.9, 127.8, 127.7, 127.6, 127.3, 127.1, 109.2, 98.9, 73.5, 72.9, 72.7, 70.3, 67.1, 67.0, 64.1, 63.5, 52.5, 32.2, 29.2, 26.9, 25.3, 23.4; HRMS (ESI) *m*/*z* calcd for C₃₉H₄₉NO₁₀Na [M⁺Na]⁺ 714.3254, found 714.3221.

### 2.8. Synthesis of 5-tert-butyl-2-methylphenyl (3,4,6-tri-O-acetyl-2-azido-2-deoxy-α-D-glucopyranosyl)-(1→2)-3,7-di-O-benzoyl-4-O-para-bromobenzyl-6-O-benzy)-1-thio-L-g/ycero-D-manno-heptopyranoside 30

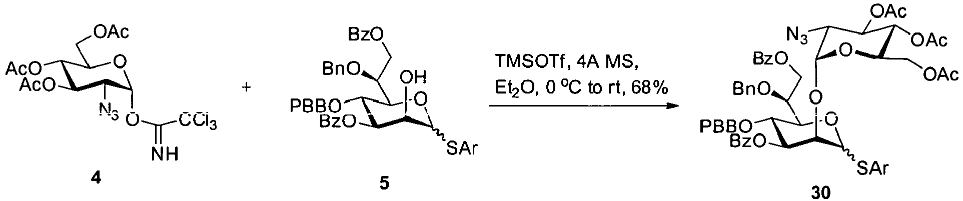

To a stirred mixture of the donor 4 (70 mg, 0.147 mmol), acceptor **5** (28 mg, 0.033 mmol), and freshly activated 4Å MS in dry Et₂O (3 mL) at 0 °C, was added dropwise TMSOTf in CH₂Cl₂ (0.05 M, 0.34 mL) under nitrogen. After 0.5 h, the temperature was allowed to warm up naturally to room temperature and the stirring continued for overnight. The mixture was then filtered and concentrated *in vacuo.* The residue was purified silica gel column chromatography (hexane/EtOAc: 7/1 to 6/1) provided **30** (26 mg, 68%) as a white solid: [α]²⁰_{D} = +78.61 (c 0.8, CHCl₃); ¹H NMR (400 MHz, CDCl₃) δ 8.07 - 7.91 (m, 4 H), 7.58 - 7.21 (m, 14 H), 7.16 (dd, *J* = 2.0, 8.0 Hz, 1 H), 7.08 - 6.85 (m, 3 H), 5.83 (d, *J* = 2.0 Hz, 1 H), 5.66 (dd, *J* = 9.2, 10.4 Hz, 1 H), 5.58 (m, 1 H), 5.07 (dd, *J* = 9.2, 10.4 Hz, 1 H), 5.05 (d, *J* = 3.2 Hz, 1 H), 4.85 (d-like, *J* = 12.0 Hz, 1 H), 4.77 (dd, *J* = 5.6, 10.8 Hz, 1 H), 4.58 (m, 2 H), 4.55 (m, 1 H), 4.42 (m, 3 H), 4.32 (m, 2 H), 4.17 (t, *J* = 6.8 Hz, 1 H), 3.98 (m, 2 H), 3.41 (dd, *J* = 3.6, 10.4 Hz, 1 H), 2.36 (s, 3 H), 2.13 (s, 3 H), 1.95 (s, 3 H), 1.85 (s, 3 H), 1.28 (s, 9 H); ¹³C NMR (100 MHz, CDCl₃) δ 170.5, 169.8, 169.7, 166.0, 165.9, 150.0, 137.9, 136.9, 135.7, 133.4, 133.0, 132.6, 131.2, 130.2, 129.7, 129.6, 129.5, 129.3, 129.0, 128.7, 128.5, 128.3, 128.1, 127.9, 124.9, 121.3, 99.1, 85.9, 73.3, 73.1, 72.6, 72.2, 71.7, 70.7, 70.5, 68.6, 68.4, 68.2, 62.6, 61.6, 61.2, 34.5, 31.3, 20.7, 20.5, 20.3, 20.2; HRMS (ESI) *m*/*z* calcd for C₅₈H₆₂BrSN₃O₁₅Na [M+Na]⁺ 1176.2962, found 1176.2992.

### 2.9. Synthesis of N-Phenyl Trifluoroacetimidate (3,4,6-tri-O-acetyl-2-azido-2-deoxy-α-D-glucopyranosyl)-(1→ 2)-3,7-di-O-benzoyl-4-O-para-bromobenzyl-6-O-benzyl-L-glycero-D-manno-heptopyranoside 2

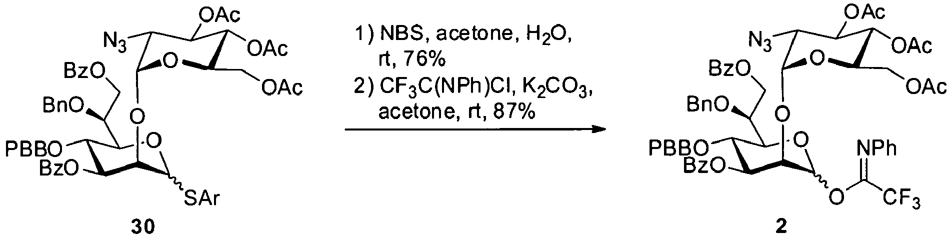

To a solution of compound **30** (83 mg, 0.072 mmol) in acetone/H₂O (10/1, v/v, 2.2 mL), was added NBS (38 mg, 0.22 mmol). After being stirred at room tempearature for 1 h, the mixture was diluted with EtOAc, washed with saturated aqueous NaHCO₃ and brine. The organic layer was dried over Na₂SO₄, filtered, and concentrated *in vacuo*. The residue was purified by silica gel column chromatography (hexane/EtOAc: 2/1) to afford the corresponding hemiacetal (54 mg, 76%) as a colorless syrup. To a solution of the above hemiacetal (69 mg, 0.070 mmol) and K₂CO₃ (27 mg, 0.195 mmol) in acetone (1.5 mL), was added 2,2,2-trifluoro-N-phenylacetimidoyl chloride (100 mg, 0.482 mmol). After being stirred at room temperature for 30 min, the solution was filtered and concentrated *in vacuo* to a residue, which was purified by silica gel column chromatography (hexane/EtOAc: 3/1) to afford **2** (70 mg, 87%) as a colorless syrup: [α]²⁰D = +47.32 (c 0.8, CHCl₃); ¹H NMR (400 MHz, CDCl₃) δ 8.06 - 8.01 (m, 4 H), 7.61 - 7.09 (m, 18 H), 6.86 - 6.81 (m, 4 H), 5.62 (dd, *J* = 9.2, 10.4 Hz, 1 H), 5.59 (dd, *J* = 2.8, 9.2 Hz, 1 H), 5.05 (dd, *J* = 9.6, 10.4 Hz, 1 H), 4.85 (d-like, *J* = 12.0 Hz, 1 H), 4.77 (dd, *J* = 5.2, 10.8 Hz, 1 H), 4.60 (d-like, *J* = 12.4 Hz, 1 H), 4.49 - 4.40 (m, 5 H), 4.27 (dd, *J* = 4.0, 12.4 Hz, 1 H), 4.15 (t, *J* = 6.4 Hz, 1 H), 4.08 (d-like, *J* = 9.6 Hz, 1 H), 3.99 (d-like, *J* = 12.4 Hz, 1 H), 3.40 (dd, *J* = 4.0, 10.8 Hz, 1 H), 2.12 (s, 3 H), 2.01 (s, 3 H), 1.95 (s, 3 H); ¹³C NMR (100 MHz, CDCl₃) δ 170.5, 169.8, 169.7, 166.2, 165.9, 143.2, 137.8, 136.7, 133.6, 133.3, 131.4, 129.8, 129.7, 129.6, 129.3, 129.2, 128.9, 128.8, 128.6, 128.5, 128.2, 128.1, 124.6, 121.6, 119.4, 99.3, 77.3, 73.6, 73.5, 72.7, 72.5, 72.2, 70.4, 68.5, 68.2, 62.3, 61.5, 61.1, 20.8, 20.7, 20.6; HRMS (ESI) *m*/*z* calcd for C₅₅H₅₂BrF₃N₄O₁₆Na [M+Na]⁺ 1185.2400, found 1185.2423.

### 2.10.Synthesis of methyl [N-benzyl-benzyloxycarbonyl-5-aminopentyl (2,7-di-O-acetyl-3-O-levulinoyl-4-O-para-bromobenzyl-6-O-benzyl-L-glycero-α-D-manno-heptopyranosyl)-(1→5)-4-O-benzyl-7,8-O-isopropylidene-3-deoxy-α-D-manno-oct-2-ulopyranosid]onate 31

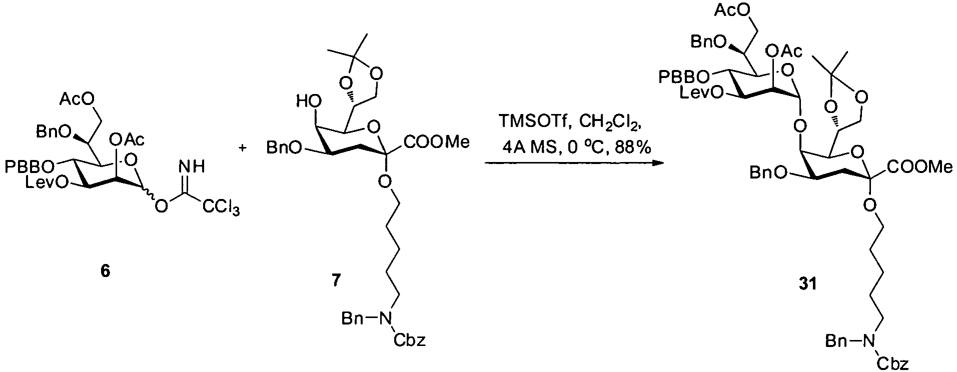

To a stirred mixture of the donor **6** (100 mg, 0.126 mmol), acceptor **7** (65 mg, 0.094 mmol), and freshly activated 4Å MS in dry CH₂Cl₂ (5.5 mL) at 0 °C, was added dropwise TMSOTf in CH₂Cl₂ (0.05 M, 138 µL) under nitrogen. After being stirred at 0 °C for 1 h, the mixture was quenched with Et₃N, filtered and concentrated *in vacuo.* The residue was purified by silica gel column chromatography (cyclohexane/EtOAc: 2/1 to 3/2) to afford **31** (110 mg, 88%) as a colorless syrup: [α]²⁰_{D} = +35.1 (c 1.0, CHCl₃); ¹H NMR (400 MHz, CDCl₃) δ 7.45 - 7.05 (m, 24 H), 5.40 (dd, *J* = 3.2, 9.6 Hz, 1 H), 5.33 (dd, *J* = 2.0, 3.2 Hz, 1 H), 5.19 (d, *J* = 1.6 Hz, 1 H), 5.16 (m, 2 H), 4.76 (d-like, *J* = 12.0 Hz, 1 H), 4.64 (d-like, *J* = 11.6 Hz, 1 H), 4.50 (m, 3 H), 4.42 (m, 2 H), 4.33 (m, 2 H), 4.19 (m, 3 H), 4.09 (br s, 1 H), 4.01 (t, *J* = 9.6 Hz, 1 H), 3.92 (dd, *J* = 2.8, 12.4 Hz, 1 H), 3.86 (m, 1 H), 3.77 (m, 1 H), 3.76 (s, 3 H), 3.65 (m, 1 H), 3.25 (m, 5 H), 2.67 (m, 2 H), 2.43 (m, 2 H), 2.30 (dd, *J* = 3.6, 12.4 Hz, 1 H), 2.15 (s, 3 H), 2.11 (s, 3 H), 2.00 (t, *J* = 12.0 Hz, 1 H), 1.95 (s, 3 H), 1.49 (m, 4 H), 1.25 (s, 3 H), 1.24 (s, 3 H), 1.23 (m, 2 H); ¹³C NMR (100 MHz, CDCl₃) δ 206.2, 171.5, 170.4, 170.0, 168.4, 138.5, 137.7, 137.5, 131.3, 128.9, 128.5, 128.4, 128.3, 128.2, 128.1, 127.9, 127.8, 127.7, 127.6, 127.5, 127.2, 121.3, 109.6, 98.8, 97.4, 77.2, 74.5, 74.4, 73.2, 73.0, 72.6, 72.3, 72.2, 72.1, 71.9, 71.7, 70.2, 70.0, 67.9, 67.1, 66.3, 63.5, 52.4, 37.8, 31.8, 29.8, 29.7, 29.3, 27.9, 26.8, 24.7, 23.4, 22.7, 21.0, 20.9; HRMS (ESI) *m*/*z* calcd for C₆₉H₈₂BrNO₂₀Na [M+Na]⁺ 1346.4511, found 1346.4506.

### 2.11.Synthesis of methyl [N-benzyl-benzyloxycarbonyl-5-aminopentyl (2,7-di-O-acetyl-4-O-para-bromobenzyl-6-O-benzyl-L-glycero-α-D-manno-heptopyranosyl)-(1→5)-4-O-benzyl-7,8-O-isopropylidene-3-deoxy-α-D-manno-oct-2-ulopyranosid]onate 3

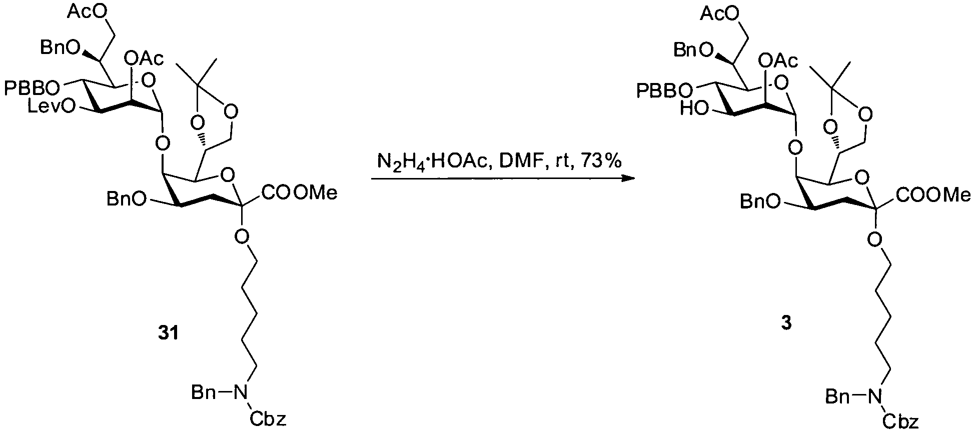

To a solution of **31** (105 mg, 0.079 mmol) in DMF (3 mL) at room temperature, was added hydrazine acetate (30 mg, 0.324 mmol). After being stirred at room temperature for 40 min, the mixture was diluted with EtOAc, washed with saturated aqueous NaHCO₃, and brine. The organic layer was dried over Na₂SO₄, filtered, and concentrated *in vacuo.* The residue was purified by silica gel column chromatography (hexane/EtOAc: 1/1) to give **3** (71 mg, 73 %) as a colorless syrup: [α]²⁰_{D} = +28.6 (c 0.8, CHCl₃); ¹H NMR (400 MHz, CDCl₃) δ 7.47 - 7.10 (m, 24 H), 5.26 (d, *J* = 1.2 Hz, 1 H), 5.20 (m, 3 H), 4.76 (d-like, *J* = 12.0 Hz, 2 H), 4.65 (d-like, *J* = 11.6 Hz, 1 H), 4.50 - 4.43 (m, 4 H), 4.38 - 4.20 (m, 6 H), 4.11 (m, 2 H), 3.95 (dd, *J* = 2.4, 12.0 Hz, 1 H), 3.83 (m, 2 H), 3.76 (s, 3 H), 3.68 (m, 1 H), 3.35 (m, 2 H), 3.25 (m, 3 H), 2.32 (dd, *J* = 4.0, 12.8 Hz, 1 H), 2.13 (s, 3 H), 1.96 (s, 3 H), 1.94 (t, *J* = 12.0 Hz, 1 H), 1.51 (m, 4 H), 1.27 (s, 3 H), 1.26 (s, 3 H), 1.25 (m, 2 H); ¹³C NMR (100 MHz, CDCl₃) δ 171.1, 170.6, 170.5, 168.5, 138.5, 137.9, 137.8, 137.7, 131.4, 131.2, 129.0, 128.6, 128.5, 128.4, 128.2, 128.0, 127.9, 127.8, 127.7, 127.6, 127.5, 127.3, 127.1, 121.3, 109.6, 98.8, 97.0, 77.2, 75.7, 74.8, 74.5, 73.4, 72.7, 72.6, 72.2, 72.1, 71.6, 70.6, 70.2, 67.9, 67.1, 66.8, 63.6, 60.4, 52.5, 31.9, 29.2, 26.9, 24.8, 23.4, 21.1, 21.0; HRMS (ESI) *m*/*z* calcd for C₆₄H₇₆BrNO₁₈Na [M+Na]⁺ 1250.4123, found 1250.4144.

### 2.12. Synthesis of methyl [N-benzyl-benzyloxycarbonyl-5-aminopentyl (3,4,6-tri -O-acetyl-2-azido-2-deoxy-α-D-glucopyranosyl)-(1→2)-(3,7-di-O-benzoyl-4-O-para-bromobenzyl-6-O-benzy)-1-thio-L-glycero-α-D-manno-heptopyranosyl)-(1→3)-(2,7-di-O-acety)-4-O-para-bromobenzy)-6-O-benzyl-L-glycero-α-D-manno-heptopyranosyl)-(1→5)-4-O-benzyl-7,8-O-isopropylidene-3-deoxy-α-D-manno-oct-2-ulopyranosid]onate 32

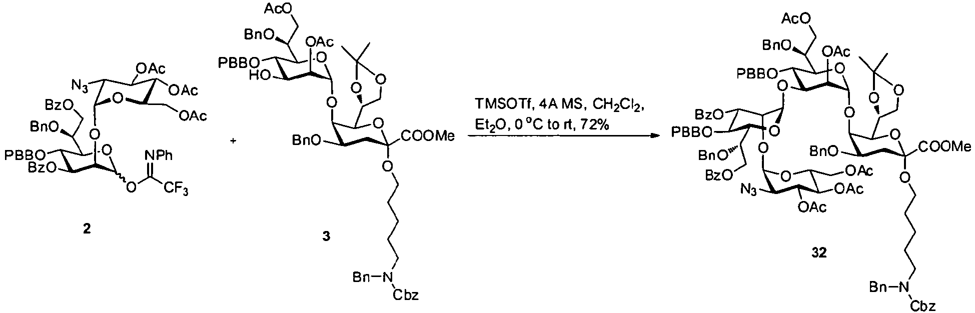

To a stirred mixture of the disaccharide donor **2** (49 mg, 42 µmol), disaccharide acceptor **3** (38 mg, 31 µmol), and freshly activated 4Å MS in dry diethyl ether and dichloromethane (1/1, v/v, 3.6 mL) at 0 °C, was added TMSOTf in CH₂Cl₂ (0.05 M, 90 dichloromethane (1/1, v/v, 3.6 mL) at 0 °C, was added TMSOTf in CH₂Cl₂(0.05 M, 90 µL) under nitrogen. The temperature was allowed to warm up naturally to room temperature and the stirring continued for 1 h. The mixture was quenched with Et₃N, and filtered. The filtrate was concentrated *in vacuo* to give a residue, which was purified by silica gel column chromatography (hexane/EtOAc/TEA: 5/2/0.07) to afford **32** (49 mg, 72%) as a white solid: [α]²⁰_{D} = +19.3 (c 1.0, CHCl₃); ¹H NMR (600 MHz, Pyridine-d₅) δ 8.49 - 8.33 (m, 4 H), 7.85 (m, 2 H), 7.66 - 7.31 (m, 33 H), 7.16 (d, *J* = 7.8 Hz, 2 H), 6.96 (d, *J* = 7.8 Hz, 2 H), 6.11 (dd, *J* = 2.4, 9.0 Hz, 1 H), 6.04 (m, 1 H), 6.02 (br s, 1 H), 5.84 (br s, 1 H), 5.50 (t, *J* = 9.6 Hz, 1 H), 5.45 - 5.37 (m, 3 H), 5.20 (m, 2 H), 5.02 (m, 4 H), 4.93 - 4.83 (m, 7 H), 4.77 (d-like, *J* = 11.4 Hz, 2 H), 4.73 - 4.62 (m, 6 H), 4.56 (m, 3 H), 4.48 (m, 1 H), 4.43 (m, 2 H), 4.28 (d-like, *J* = 11.4 Hz, 1 H), 4.18 (m, 2 H), 4.07 - 4.01 (m, 3 H), 3.91 (m, 1 H), 3.89 (s, 3 H), 3.76 (m, 1 H), 3.42 (m, 3 H), 3.29 (m, 1 H), 2.62 (d-like, *J* = 9.6 Hz, 1 H), 2.32 (t, *J* = 12.0 Hz, 1 H), 2.27 (s, 3 H), 2.16 (s, 3 H), 2.15 (s, 3 H), 2.01 (s, 3 H), 1.99 (s, 3 H), 1.55 (m, 4 H), 1.41 (s, 3 H), 1.35 (s, 3 H), 1.26 (m, 2 H); ¹³C NMR (150 MHz, Pyridine-*d₅*) δ 171.1, 171.0, 170.9, 170.7, 170.4, 169.1, 167.2, 166.7, 150.7, 140.2, 139.4, 139.3, 139.0, 138.9, 138.5, 138.3, 136.3, 134.1, 134.0, 132.3, 131.8, 131.2, 130.9, 130.8, 130.5, 130.3, 129.7, 129.6, 129.5, 129.4, 129.3, 129.2, 129.1, 128.7, 128.6, 128.2, 128.0, 124.3, 123.6, 122.1, 121.7, 110.4, 101.1, 99.8, 98.1, 76.7, 76.1, 75.3, 75.0, 74.5, 73.7, 73.5, 73.2, 73.1, 72.5, 72.3, 71.6, 70.9, 69.5, 69.4, 68.5, 67.7, 67.6, 67.3, 64.4, 62.7, 62.3, 54.7, 53.0, 51.2, 50.8, 47.9, 47.0, 30.5, 30.0, 27.4, 25.6, 24.1, 23.4, 21.5, 21.3, 21.1, 21.0, 20.9; HRMS (ESI) *m*/*z* calcd for C₁₁₁H₁₂₂Br₂N₄O₃₃Na [M⁺Na]⁺ 2222.6275, found 2222.6349.

### 2.13. Synthesis of methyl [N-benzyl-benzyloxycarbonyl-5-aminopentyl (3,4,6-tri -O-acety)-2-N-acetyl-2-deoxy-α-D-glucopyranosyl)-(1→2)-(3,7-di-O-benzoyl-4-O-para-bromobenzyl-6-O-benzyl-L-glycero-α-D-manno-heptopyranosyl)-(1→3)-(2,7-di-O-acetyl-4-O-para-bromobenzyl-6-O-benzyl-L-glycero-α-D-manno-heptopyranosyl)-(1→5)-4-O-benzyl-7,8-O-isopropylidene-3-deoxy-α-D-manno-oct-2-ulopyranosid]onate 33

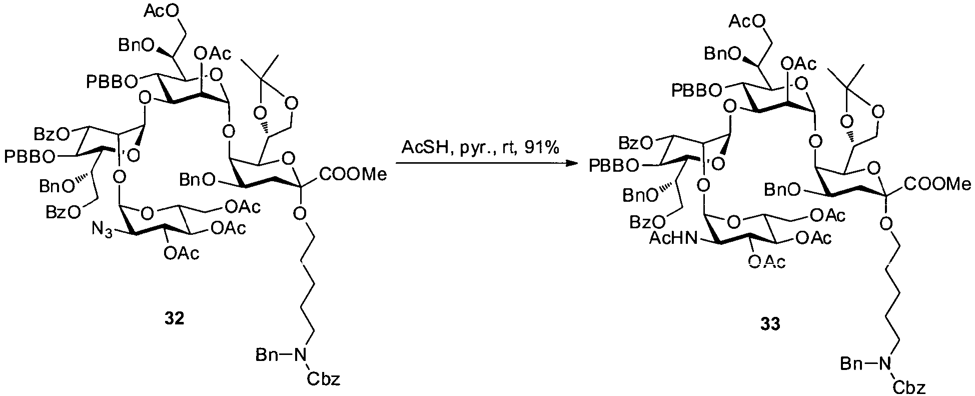

To a solution of compound **32** (37 mg, 0.017 mmol) in dry pyridine (0.3 mL), was added thioacetic acid (0.3 mL, 4.18 mmol). After being stirred at room temperature for 24 h, the solution was coevaporated with toluene to give a residue, which was purified by silica gel column chromatography (hexane/EtOAc/TEA: 3/2/0.05 to 1/1/0.02) to give **33** (34 mg, 91%) as a pale yellow solid: [α]²⁰_{D} = +26.1 (c 0.8, CHCl₃); ¹H NMR (400 MHz, Pyridine-d₅) δ 8.51 - 8.19 (m, 4 H), 7.79 - 7.18 (m, 37 H), 6.99 (d, *J* = 8.4 Hz, 2 H), 6.12 (m, 2 H), 6.04 (br s, 1 H), 5.88 (m, 1 H), 5.83 (br s, 1 H), 5.56 (t, *J* = 10.0 Hz, 1 H), 5.39 (m, 4 H), 5.24 (d-like, *J* = 12.0 Hz, 1 H), 5.05 - 4.89 (m, 9 H), 4.86 - 4.68 (m, 8 H), 4.64 - 4.54 (m, 6 H), 4.39 (m, 2 H), 4.17 (m, 2 H), 4.07 (m, 2 H), 4.00 (m, 2 H), 3.93 (s, 3 H), 3.75 (d-like, *J* = 8.8 Hz, 1 H), 3.46 (m, 3 H), 3.29 (m, 1 H), 2.63 (m, 1 H), 2.28 (s, 3 H), 2.25 (m, 1 H), 2.23 (s, 3 H), 2.02 (s, 3 H), 1.98 (s, 3 H), 1.97 (s, 6 H), 1.55 (m, 4 H), 1.41 (s, 3 H), 1.34 (s, 3 H), 1.25 (m, 2 H); ¹³C NMR (100 MHz, Pyridine-d₅) δ 171.6, 171.1, 171.0, 170.4, 170.3, 169.0, 167.3, 166.2, 140.2, 139.5, 139.4, 138.9, 138.6, 138.3, 134.4, 134.0, 132.4, 131.9, 131.2, 130.8, 130.5, 130.4, 130.2, 129.7, 129.6, 129.5, 129.4, 129.3, 129.2, 128.7, 128.6, 128.3, 128.1, 128.0, 122.2, 110.4, 100.6, 99.8, 98.2, 76.0, 75.3, 74.8, 73.7, 73.5, 73.1, 72.5, 72.0, 70.9, 69.7, 68.5, 67.7, 67.2, 64.4, 62.9, 60.7, 53.5, 53.0, 51.2, 50.8, 48.0, 47.1, 30.5, 30.0, 27.4, 25.7, 24.1, 22.8, 21.6, 21.4, 21.3, 21.2, 21.1, 20.9; HRMS (ESI) *m*/*z* calcd for C₁₁₃H₁₂₆Br₂N₂O₃₄Na [M+Na]⁺ 2238.6476, found 2238.6545.

### 2.14. Synthesis of 2-N-acetyl-2-deoxy-α-D-glucopyranosyl-(1→2)-L-glycero-α-D-manno-heptopyranosy)-(1→3)-L-glycero-α-D-manno-heptopyranosyl-(1→5)- 2-(5-amino)pentyl-3-deoxy-α-D-manno-oct-2-ulopyranosidonic acid 1

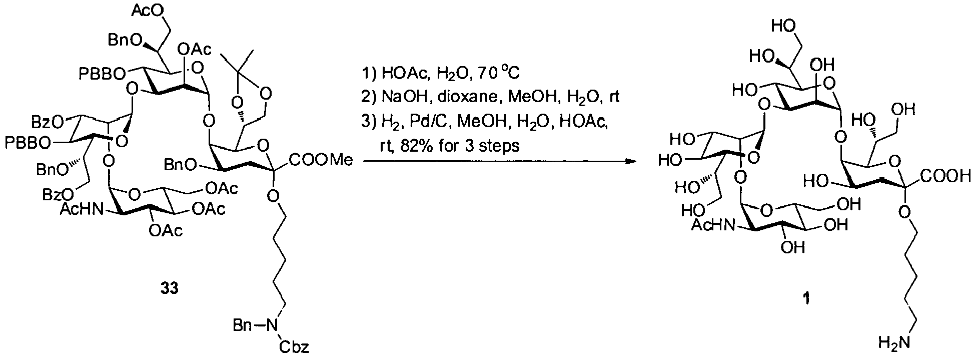

A solution of compound **33** (35 mg, 0.016 mmol) in acetic acid/water (8/1, v/v, 1.80 mL) was stirred at 70 °C for overnight. TLC indicated complete conversion of starting material. The mixture was covaporated with toluene and dried *in vacuo* to give the corresponding diol as a pale yellow syrup. The above diol was dissolved in a mixture of dioxane, methanol and 1 M aq NaOH (3/1/1, v/v/v, 1.25 mL). After being stirred at room temperature for overnight, the reaction mixture was diluted with methanol and neutralized with Amberlite IR120 H⁺ resin. After filtration, the filtrate was concentrated *in vacuo* to give the corresponding tetrasaccharide as a white solid. A mixture of the above tetrasaccharide and Pd/C (70 mg, 10%) in methanol, water and acetic acid (50/25/1, v/v/v, 3.04 mL) was stirred under an atmosphere of H₂ at room temperature for 24 h. Filtration, concentration *in vacuo* and elution through Sephadex LH-20 column (H₂O) provided **1** (12 mg, 82% for 3 steps) as a white solid: [α]²⁰_{D} = +64.2 (*c* 0.3, H₂O); ¹H NMR (600 MHz, D₂O) δ 5.42 (br s, 1 H), 5.12 (d, *J* = 3.6 Hz, 1 H), 5.09 (d, *J* = 1.2 Hz, 1 H), 4.16 (m, 2 H), 4.09 (m, 2 H), 4.06 - 4.01 (m, 4 H), 3.98 (m, 3 H), 3.95 (dd, *J* = 3.0, 12.0 Hz, 1 H), 3.91 (dd, *J* = 3.6, 10.8 Hz, 1 H), 3.85 - 3.76 (m, 6 H), 3.73 - 3.63 (m, 6 H), 3.49 (t, *J* = 9.6 Hz, 1 H), 3.44 (m, 1 H), 3.30 (m, 1 H), 3.01 (t, *J* = 7.8 Hz, 2 H), 2.10 (dd, *J* = 4.8, 12.6 Hz, 1 H), 2.07 (s, 3 H), 1.84 (t, *J* = 12.6 Hz, 1 H), 1.69 (m, 2 H), 1.62 (m, 2 H), 1.44 (m, 2 H); ¹³C NMR (150 MHz, D₂O) δ 175.0, 174.4, 101.3, 100.3, 99.7, 99.2, 78.6, 76.7, 75.0, 72.2, 71.9, 71.6, 71.3, 70.6, 70.4, 70.1, 69.9, 69.2, 68.7, 68.5, 66.3, 65.9, 65.6, 63.3, 63.2, 62.9, 60.4, 53.9, 39.3, 34.8, 28.1, 26.4, 22.4, 21.9; HRMS (ESI) *m*/*z* calcd for C₃₅H₆₁N₂O₂₅ [M-H]⁻ 909.3563, found 909.3629.

## Claims

1. Synthesis of the tetrasaccharide **1** of the chemical formula 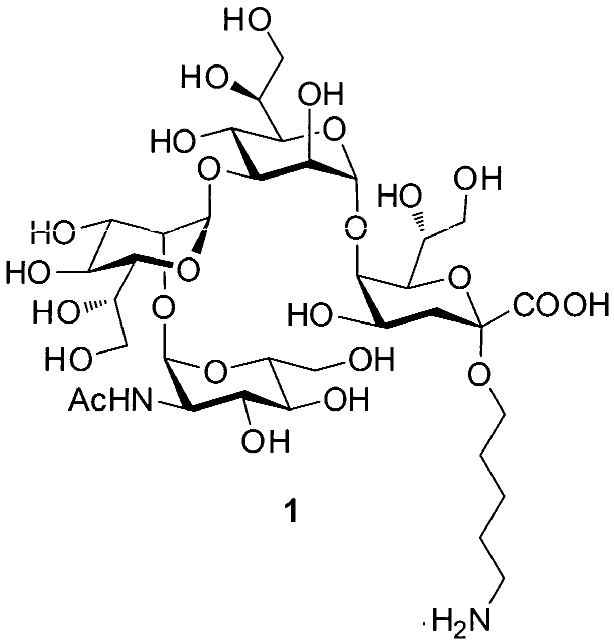 comprising the steps:
A) Reacting the compound **6*** of the formula 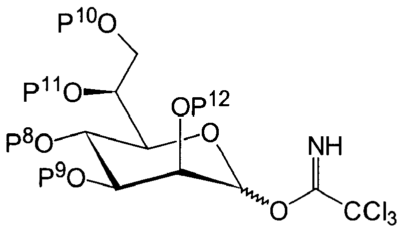 wherein
P⁸ - P¹² represent protecting groups
with the compound 7* of the formula 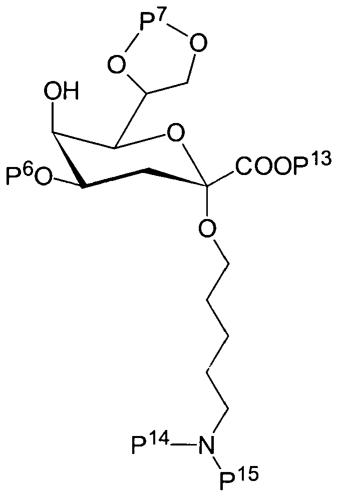 wherein
P⁶, P⁷ P¹³ - P¹⁵ represent protecting groups
in order to obtain compound **31*** of the following chemical formula 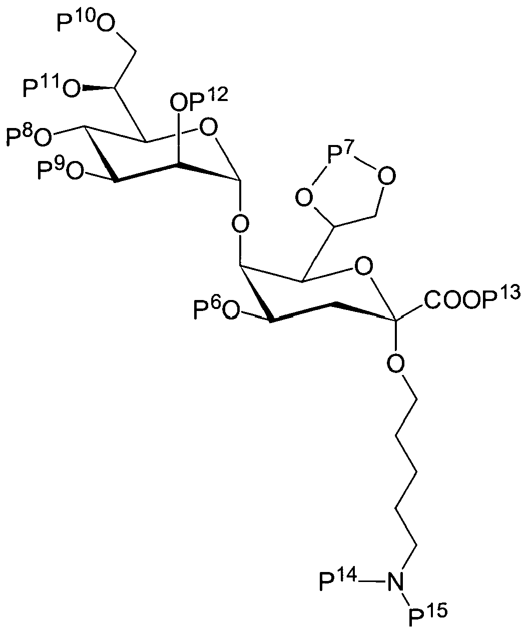 wherein the protecting group P⁹ is selectively cleaved in order to obtain compound **3***.
B) Reacting the compound **4*** of the formula 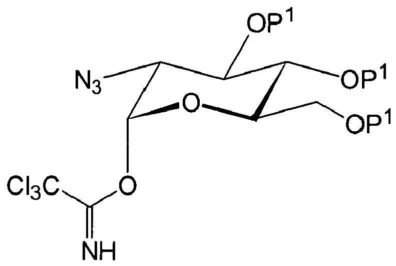 wherein
the groups P¹ represent the same protecting group
with the compound **5*** of the following chemical formula 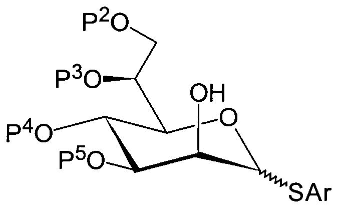 wherein
P² - P⁵ represent protecting groups and
A*r* represents an aromatic ring or aromatic ring system
in order to obtain compound **30*** of the following chemical formula 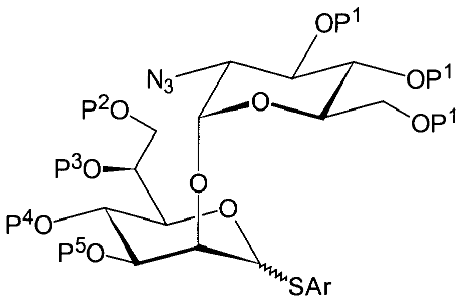 wherein
the group -SAr is converted to the group -O-C(=NPh)-CF₃ in order to obtain compound **2*** of the following chemical formula 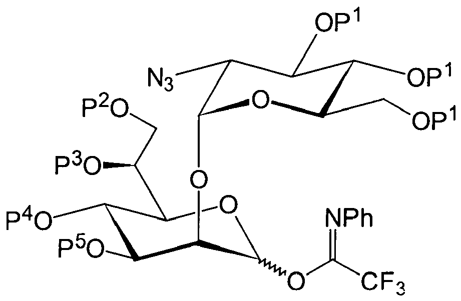
C) Reacting the compound **2*** with compound **3*** of the formula 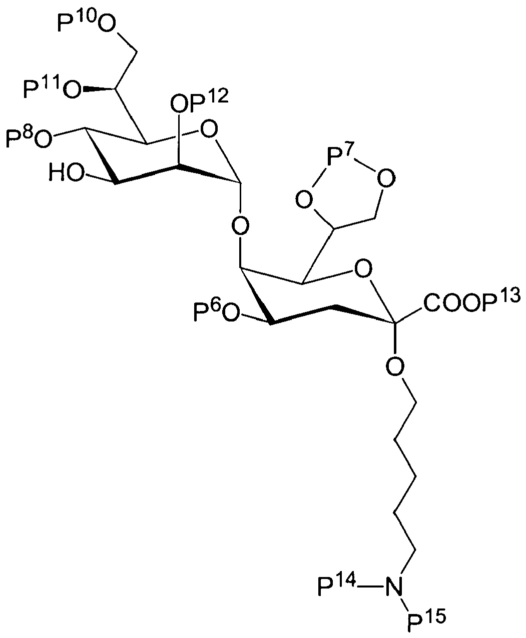 in order to obtain compound **32*** wherein the azide group is converted into an acetamidegroup and wherein the protecting groups P¹ - P⁸ and P¹⁰ - P¹⁵ are cleaved in order to obtain compound **1**.

2. Synthesis according to claim 1 comprising step D:
D) preparing a salt of compound **1** or preparing a lyophilisate of compound **1** or of the salt of compound **1.**

3. Synthesis according to claim 1, wherein the reaction of compound **6*** and **7*** is performed in a polar aprotic solvent using TMSOTf.

4. Synthesis according to claim 1, wherein the reaction of compound **4*** and **5*** is performed in an aprotic solvent using TMSOTf.

5. Synthesis according to claim 1, wherein the conversion of compound **30*** to **2*** is performed in two steps, first by reacting compound **30*** in a polar aprotic solvent and water mixture using NBS and second by reacting the product obtained after the first step with CF₃C(=NPh)Cl and a base in a polar aprotic solvent.

6. Synthesis according to claim 1, wherein the conversion of compound **31*** to **3*** is performed in a polar aprotic solvent by means of hydrazine or a hydrazinium salt.

7. Synthesis according to claim 1, wherein the reaction of compound **2*** and **3*** is performed in an aprotic solvent using TMSOTf.

8. Synthesis according to claim 1, wherein the conversion of compound **32*** to **33*** is performed in a polar aprotic solvent to which a base is added or in a polar aprotic basic solvent using thioacetic acid.

9. Synthesis according to claim 1, wherein the conversion of compound **33*** to **1** is performed in three steps, first the acid-labile protecting groups are cleaved in a mixture of an acid in water; second the base-labile protecting groups are cleaved in a polar aprotic solvent or a polar aprotic solvent mixture using a base; and third the protecting groups sensitive for hydrogenation are cleaved by means of hydrogen and a catalyst.

10. The tetrasaccharide **1** of the chemical formula: 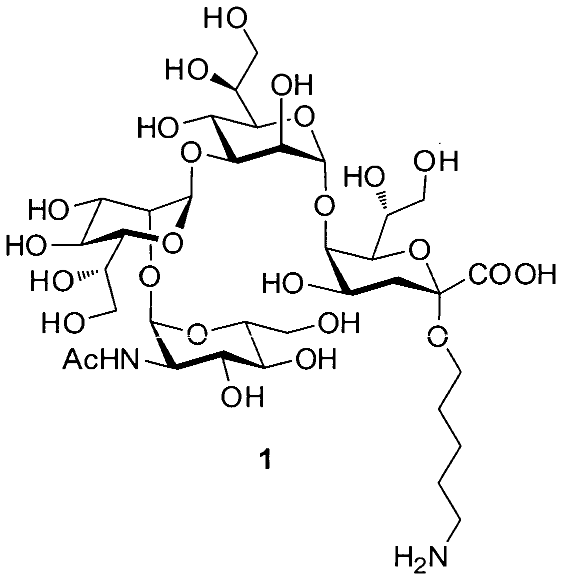 for use as a vaccine for immunization against diseases caused by bacteria containing or comprising the tetrasaccharide 1.

11. Use of the tetrasaccharide 1 according to claim 10 as a vaccine for eliciting antibodies.

12. Use of the tetrasaccharide **1** according to claim 10 as a marker in immunological assays for diagnostics of diseases caused by bacteria containing the tetrasaccharide **1**.

13. Use of the tetrasaccharide 1 according to claim 10, wherein the bacteria containing the tetrasaccharide **1** are selected from the group consisting of all strains of *Neisseria meningitidis,* wherein the LPS immunotypes are L1, L2, L3, L4, L5, L6, L7, L8, L9 and/or L11.

14. Use of the tetrasaccharide **1** according to claim 10, wherein the diseases caused by bacteria containing the tetrasaccharide **1** are selected from the group consisting of meningitis, septicemia, pneumonia and nasopharyngitis.

15. The tetrasaccharide **1** of the chemical formula: 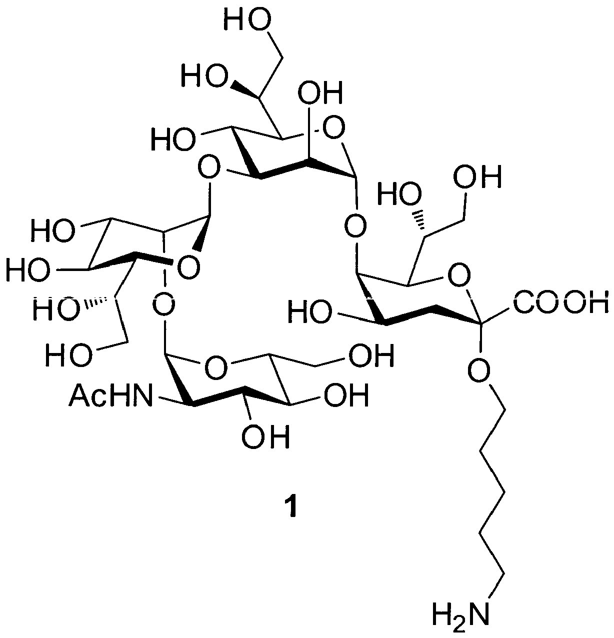 not contaminated with PEA and endotoxin lipid A.

16. The tetrasaccharide **1** according to claim 15 which does not have any microheterogenicity and/or which is at least 95% pure.

17. Pharmaceutical composition containing the tetrasaccharide **1** according to claim 15 or 16 together with at least one pharmaceutically acceptable carrier, cryoprotectant, lyoprotectant, excipient and/or diluent.
